# EUROPEAN PATENT APPLICATION

(11) **EP 3 973 949 A1**
(43) Date of publication of application: **30.03.2022**
(21) Application number: 20198425.9
(22) Date of filing: 25.09.2020
(51) Int. Cl.: A61K 9/00, A61K 47/14, A61K 47/44, A61P 3/02

(54) **STABILIZATION OF VITAMIN A IN A NUTRITIONAL SOLUTION**

(71) Applicant: Baxter International Inc, Deerfield, IL 60015 (US); BAXTER HEALTHCARE SA, 8152 Glattpark (Opfikon) (CH)
(72) Inventor: BOUREZG, Zouaoui, 1420 Braine l Alleud (BE); DUPONT, Caroline, 1050 Ixelles (BE); BROSSARD, Gabriel, 1190 Forest (BE); TROUILLY, Jean-Luc, 1420 Braine l´Alleud (BE); COUTHOUIS, Aurélie, B-1480 Tubize (BE)
(74) Representative: Hertin und Partner Rechts- und Patentanwälte PartG mbB

(57) **Abstract**

The invention relates to a medical product for preventing or correcting vitamin A deficiency in a patient comprising a lipid emulsion in a flexible container, the lipid emulsion comprising (a) vitamin A in the lipid phase of the lipid emulsion, (b) optionally additionally vitamin D, vitamin E and/or vitamin K in the lipid phase of the lipid emulsion, and (c) no more than 1.5 ppm dissolved oxygen (DO), (d) wherein the pH of the lipid emulsion is from 5 to 9.

## Description

The invention relates to the field of clinical nutrition, corresponding medical products and nutritional solutions.

The invention relates to a medical product for preventing or correcting vitamin A deficiency in a patient comprising a lipid emulsion in a flexible container, the lipid emulsion comprising (a) vitamin A, preferably in the form of retinyl palmitate, in the lipid phase of the lipid emulsion, (b) optionally additionally vitamin D, vitamin E and/or vitamin K in the lipid phase of the lipid emulsion, and (c) no more than 1.5 ppm dissolved oxygen (DO), (d) wherein the pH of the lipid emulsion is from 5 to 9.

In certain embodiments of the invention, the solution of the medical product of the invention comprises vitamin A in form of retinyl palmitate, wherein the vitamin A is preferably provided in a lipid emulsion wherein the lipid component is an oil with a low peroxide value. The lipid emulsion may be ready-to-use and may be contained in one chamber of a large-volume multi chamber container having at least two, at least three, at least four, at least five or at least six chambers. Furthermore, the invention relates to a method of preparing a medical product of the invention.

### BACKGROUND OF THE INVENTION

Parenteral Nutrition aims to supply nutrients to patients by venous access. Nutrients are composed of macronutrients (lipids, amino acids or proteins and dextrose or carbohydrates), micronutrients (vitamins and trace elements) and electrolytes.

Parenteral nutrition, such as in the form of one or more solutions, can be provided in the form of flexible bags, either in the form of single flexible bags containing glucose, amino acids or lipids with or without electrolytes, which can be mixed together prior administration, or multi chamber flexible bags providing separated macronutrients and electrolytes, in a ready to use format. The bags are typically made of a synthetic or plastic material, for example materials such as polypropylene (PP), polyethylene (PE), ethylene vinyl alcohol (EVOH), ethylene-vinyl acetate (EVA) and all possible copolymers, essentially any synthetic material suitable for containing the components to be administered.

In the state of the art, micronutrients are typically added to nutrition bags directly before administration. For this purpose, vitamins can be provided in glass vials in the form of lyophilizates or solutions to be reconstituted and/or mixed into the nutrition/infusion bags. Trace elements are also provided in glass vials or polypropylene ampules meant to be mixed into infusion bags prior to administration.

Prior to usage, referring to the start administering the formulation to the patient, the micronutrients are sometimes added to the mixture or macronutrients via an injection port of the container or bag (septum) or are added via a Y-connector to the infusion line. This process takes time and several handling steps increasing the risk of errors or contamination.

The major reason for adding reconstituted micronutrients and in particular vitamins that have been reconstituted from lyophilized formulations to macronutrients or the mixture directly before administration is that some vitamins have significant stability issues when provided in liquid ready-to-use products. Vitamin A and vitamin C are two major examples (Ferguson et al. A Review of Stability Issues Associated with Vitamins in Parenteral Nutrition; Clinical Nutrition ESPEN 2014, vol.9 issue 2). However, as vitamin A is regarded as an essential vitamin required in total PN (TPN), it must be a component of an "all-in-one" ready-to-use formulation.

Vitamin A is a generic term that refers to fat-soluble compounds found as preformed vitamin A (retinol) in animal products and as provitamin A carotenoids in fruits and vegetables. The three active forms of vitamin A in the body are retinol, retinal, and retinoic acid. Vitamin A compounds are essential fat-soluble molecules predominantly stored in the liver in the form of retinyl esters (e.g., retinyl palmitate). When appropriate, retinyl esters are hydrolysed to generate all-trans-retinol, which binds to retinol binding protein (RBP) before being released in the bloodstream. The all-trans-retinol/RBP complex circulates bound to the protein, transthyretin, which delivers all-trans-retinol to peripheral tissues.

Vitamin A is generally difficult to stabilize. For one, vitamin A, or retinol, is the most light-sensitive micronutrient found in PN admixtures besides vitamin C and vitamin B12. For example, when subjected to light in unprotected bags or administration sets, it undergoes extensive photodegradation.

Known approaches of stabilizing vitamin A compounds in a liquid preparation for PN have placed vitamin A together with the other fat-soluble vitamins in a lipid phase or, alternatively, include stabilizing vitamin A in a fat-containing lyophilized mixture with other vitamins for later addition to the PN product. Since it is known that vitamin A is light sensitive, light protection has also been used in the state of the art (e.g. in the form of an appropriate container such as brown glass or aluminium cover around a flexible bag). For example, the product Vitalipid^{®} (Fresenius Kabi) comprises vitamin A together with vitamins D₂, K₁ and E in a lipid emulsion provided in a 10mL ampoule which contains, per 1mL, 100mg soy oil, 12mg egg lecithin and 22mg glycerol. The lipid emulsion is added to parenteral nutrition formulations for injection before administration to the patient.

Furthermore, sorption of retinol may occur with bags and administration set tubing, further reducing the amount of vitamin A being administered to the patient. This problem has been much reduced through the use of the less reactive palmitate ester, rather than the acetate ester. The introduction of tubing containing polyolefine, which is free of PVC, plasticizers, adhesives or latex, has further reduced the absorption of vitamin A.

Still, these measures, when scrutinized, are not good enough to allow for a product for TPN providing all nutrients in liquid preparations in a way that no exposure of the liquids to potential sources of infection are required.

Another source of degradation of vitamins are peroxides generated by lipid emulsions. Lipid emulsions containing polyunsaturated fatty acids (PUFAs) are at an increased risk of peroxidation. Vitamin E acts as a major scavenger for free radicals and prevents lipid peroxyl radicals from reacting with fatty acid side chains. Nevertheless, peroxidation still occurs to some degree and further investigation is required to understand the relationship between the composition of lipid emulsions and the degradation of vitamins.

There have been descriptions of products including fat soluble vitamins such as vitamin A into the chamber containing the lipid formulation in order to prevent dissociation, oxidation and isomerisation. However, while such an approach may give vitamin A some minor protection, it is not enough to stabilize it efficiently for use in a PN product with a shelf-life of 12 to 24 months without the need to refridgerate or otherwise specifically treat such product(Ferguson et al. A Review of Stability Issues Associated with Vitamins in Parenteral Nutrition; Clinical Nutrition ESPEN 2014, vol.9 issue 2).

Consequently, some experts believe that the best way of reliably providing the required amounts of vitamin A in the context of TPN is the provision of a stand-alone formulation that should be combined with the other required nutrients at the time point of administration.

Accordingly, there is no medical product and in particular no TPN product available at the moment that provides prestored vitamin A in a ready to use liquid that is sufficiently stable for prolonged storage, such as 6, 12 or even 24 months at temperatures of up to 25°C or 30°C, which would be required in many countries if storage is intended at room temperature. In particular, it has so far not been possible to stabilize vitamin A in standard parenteral nutrition products which contain a variety of different compounds in flexible bags, mostly multi-chamber bags, which in addition will generally have to undergo terminal heat sterilization to be compliant with, for example, Unites States and European Pharmacopoeia requirements.

Specifically, a technical problem underlying the present invention is to provide a preferably terminally heat-sterilized pre-prepared and pre-packaged product for parenteral nutrition which can be used for preventing or correcting vitamin A deficiency in a patient comprising a ready-to-use formulation for parenteral administration provided in a flexible container comprising vitamin A or a vitamin A formulation. In particular, it is difficult to provide a multi-chamber bag comprising macronutrients selected from fats, carbohydrates and amino acids, peptides and proteins, and micronutrients comprising vitamins and trace elements, wherein the terminally heat-sterilized multi-chamber bag also stably comprises vitamin A.

Because of the above, to date there is no ready-to-use medical product for parenteral administration available that comprises a solution for parenteral administration to a patient in need thereof comprising vitamin A in a way that ensures stability over a prolonged period of time, specifically products which provide all macronutrients and potentially also vitamins and trace elements in one multi-chamber bag. Products which provide for carbohydrate and amino acid solutions in dual chamber bags and which also comprise vitamin A are known, such as, for example, Elneopa or Neoparen (both Otsuka Pharmaceutical Factory, Inc.) or Onepal (AY Pharmaceuticals Co., Ltd.). However, such products do not provide for a lipid emulsion. Ready-to-use products comprising a lipid emulsion formulation, generally in a 3-chamber container, are more complex in production and cannot be sterilized by filtration, which is generally the way to avoid the negative impact of heat on the stability of vitamin A.

In addition, such products tend to have lower total (reconstituted) volumes than those products which also comprise a lipid emulsion formulation. So, even in case of terminal heat sterilization, the required heat exposure is lower for such products than for products encompassing also said lipid emulsion formulations which potentially allows to add vitamin A to such products. Otherwise, as described before, vitamin A must be manually added to the ready-to-use parenteral nutrition formulations shortly before administration or must be administered individually, and both processes are associated with a risk of medication error, and include the significant risk of introducing contamination to the sterilized ready-made products. Contaminations can include potential infectious agents, which represent a serious risk factor to patients in hospitals, especially those receiving nutritional solutions e.g. via parenteral administration.

In light of the prior art, there remains a significant need to develop medical products for preventing or correcting vitamin A deficiency in a patient that are straightforward, have a reasonable shelf-life and are easy to use, including avoiding additional mixing steps. Due to the instability of vitamin A in solution such products are not readily available.

### SUMMARY OF THE INVENTION

In light of the prior art the technical problem underlying the present invention is to provide a preferably terminally heat sterilized pre-prepared, medical product for preventing or correcting vitamin A deficiency in a patient comprising a ready-to-use formulation for parenteral administration provided in a flexible container comprising vitamin A.

This problem is solved by the features of the independent claims. Preferred embodiments of the present invention are provided by the dependent claims.

The invention therefore relates to a medical product for preventing or correcting vitamin A deficiency in a patient comprising a lipid emulsion in a flexible container, the lipid emulsion comprising:
a. vitamin A in the lipid phase of the lipid emulsion,
b. optionally vitamin D, vitamin E and/or vitamin K in the lipid phase of the lipid emulsion, and
c. no more than 1.5 ppm dissolved oxygen (DO),
d. wherein the pH of the lipid emulsion is from 5 to 9.

In embodiments, the invention relates to a terminally heat sterilized multi-chamber container for parenteral nutrition, comprising at least one chamber comprising an amino acid formulation, a lipid emulsion formulation or a carbohydrate formulation, and at least a second formulation comprising vitamin A, wherein the vitamin A formulation
(a) is provided in a lipid emulsion;
(b) is provided optionally together with vitamins D, E and/or K;
(c) contains no more than 1.5 ppm dissolved oxygen (DO); and
(d) is provided at a pH of from 5 to 9.

According to one embodiment, such terminally heat-sterilized multi-chamber container has a reconstituted volume of at least 100 mL, of at least 250mL, of at least 500mL, of at least 800mL, of at least 1000mL, of at least 1200mL, of at least 1500mL, of at least 1800mL, of at least 2000mL, of at least 2200mL, of at least 2400mL, of at least 2600mL, of at least 2800mL or of at least 3000mL. It is a surprising finding that it is possible to provide a terminally heat sterilized multi-chamber container for parenteral nutrition which also stably comprises vitamin A. The multi-chamber container according to the embodiment of the invention preferably consists of a flexible container having two or more chambers. Importantly, the multi-chamber container of the invention underwent a terminal heat-sterilization process with an F₀ value of at least 8 minutes, which ensures a degree of sterility that makes the solution safe for parenteral administration. Accordingly, the present invention allows to provide a large volume product, such as the described multi-chamber container for parenteral nutrition, which is terminally heat-sterilized and does not require any aseptic filling step for at least one of the comprised formulations. Accordingly, the expression "medical product" as used herein, encompasses terminally heat-sterilized multi-chamber containers as described herein, wherein a vitamin A formulation as described in further detail in the present application is present in at least one of the chambers of the multi-chamber container. The use of the terms "large-volume" (multi chamber) container and (multi chamber container "high(er) volume" in the context of the invention preferably refers to a products or (multi chamber) containers of a total reconstituted volume of of at least 500mL, of at least 800mL, of at least 1000mL, of at least 1200mL, of at least 1500mL, of at least 1800mL, of at least 2000mL, of at least 2200mL, of at least 2400mL, of at least 2600mL, of at least 2800mL or of at least 3000mL.

The expression "terminally sterilized" or "terminally heat-sterilized" means that such products must have a probability of non-sterile unit (PNSU) or a sterility assurance level (SAL) of not more than one in a million units produced, in accordance with the guidelines in Europe and the United States. SAL has been defined by European Pharmacopoeia in such a way that its numerical value is the same of PNSU. Accordingly, a SAL or PNSU of 10⁻⁶ indicates that the probability of an organism surviving to the end of the sterilization process in any single unit of product is less than one in one million. The proof that a terminally sterilized product complies with the 10⁻⁶ SAL/PNSU can be accomplished by several different sterilization cycle development approaches. The proper application of this method requires extensive scientific knowledge regarding the sterilization method selected for use with a specific product. Further background information is provided, for example, in von Woedtke and Kramer, GMS KHI (2008), 3(3), 1-10 (ISSN 1863-5245). The expression "sterility" means the absence of all viable microorganisms including viruses.

The expression "reconstituted volume" as used herein refers to the total volume of all solutions contained in the multi-chamber container. The expression is not intended to be limited to refer to the total volume of the solution once the seals between the chambers have been broken and the solutions contained in the respective chambers have been combined. It also refers to the added-up volume of the solutions of the separate chambers before reconstitution.

The present invention modifies known parameters influencing vitamin A stability but also modifies additional factors that were not known to influence vitamin A stability leading to a completely unexpected medical product ensuring vitamin A stability in the lipid emulsion of the medical product of the invention. Importantly, besides including vitamin A in the lipid phase of a lipid emulsion that also comprises other fat-soluble vitamins, modification of the oxygen concentration and the pH of the emulsion were examined in order to identify the best conditions for long-term stabilization of vitamin A in the lipid emulsion.

In the context of the medical product of the invention, the specific features of the claimed lipid emulsion enables the provision of vitamin A in association with other vitamins, such as fat-soluble vitamins that are provided along vitamin A in the lipid phase of the emulsion, and water-soluble vitamins that can be provided in the aqueous phase of the emulsion, in a flexible container. Surprisingly, vitamin A in the emulsion of the medical product withstands terminal heat sterilization, even in large volume products, such as, for example, ready-to-use parenteral nutrition products consisting of three or more chambers, which due to their higher volume require a relatively high heat exposure when terminally heat sterilized. This is especially the case for PN products which provide for all macronutrient formulations, i.e. lipid emulsion, carbohydrate and amino acid formulation having total (reconstituted) volumes of about 800 mL to 2200 mL. PN products which, for example, consist of one or two chambers only, generally of an amino acid formulation and a carbohydrate formulation in a dual-chamber bag and/or which have a lower total (reconstituted) volume, especially those formulations which have a volume of less than 500, 400, 300, 200 and especially less than 100 mL, require less heat exposure during terminal heat sterilization because of their lower volume. Therefore, such products may contain some vitamin A even when heat sterilized. Also, where the products do not comprise a lipid formulation, the products can be sterilized by filtration in order to circumvent the critical heat sterilization.

In medical products of the state of the art, specifically those PN products which consist of MCBs comprising lipid emulsions and/or which have a high total (reconstituted) volume as defined before, vitamin A is prone to degradation during terminal heat sterilization due to the required heat exposure. It is an important contribution of the present invention to provide for medical products, including, but not limited to, terminally heat-sterilized ready-to-use PN products having volumes of more than 100mL, especially more than 500mL, more than 800mL, more than 1000mL, more than 1200mL and specifically more than 1500mL, which allow the stable incorporation of vitamin A. Specifically, the present invention provides for such large volume medical products which also comprise a lipid emulsion formulation, for example in multi-chamber bags for PN which comprise all macronutrients.

In the state of the art, provision of vitamin A has also been reported in the context of micellar solutions (see, for example, CN109010292B) or by adding vitamin A to mixed micelles. However, in the process of developing the present invention it was found that such liquid micelle approaches were not suitable for sustained vitamin A stabilization as required for the product of the present invention. It could be shown, in contrast, that the stable provision of vitamin A required a certain lipid emulsion and specific condition which were found in the studies made on the stability of vitamin A.

Specifically, it was found that exposure of vitamin A to an oxygen concentration of more than 1.5 ppm in the process of producing the medical product of the invention and in particular in the lipid emulsion of the medical product is detrimental for long-term stability of vitamin A and accordingly in the context of the invention exposure of the lipid emulsion and its components to a DO concentration of more than 1.5 ppm has to be avoided. Preferably, the DO concentration is below 1.0 ppm, below 0.8 ppm or even more preferably below 0.6 ppm. In further preferred embodiments, the DO concentration is below 0.5 ppm.

In the context of the invention, the concentration of DO in the lipid emulsion is no more than 1.5 ppm. Preferably, the concentration of DO in the lipid emulsion is no more than 1.0 ppm and in embodiments is no more than 0.5 ppm. It is particularly preferred that in the process of producing the lipid emulsion and preferably also in the whole manufacturing process of the medical product of the invention, it is ensured that the DO is no more than 1 ppm, preferably 0.5 ppm.

In embodiments, the DO in the lipid emulsion may be any value in the range of 0 to 1.5 ppm, such as for example about 0.001, 0.005, 0.01, 0.02, 0.03, 0.04, 0.05, 0.06, 0.07, 0.08, 0.09, 0.1, 0.12, 0.14, 0.16, 0.18, 0.2, 0.22, 0.24, 0.26, 0.28, 0.3, 0.32, 0.34, 0.36, 0.38, 0.4, 0.42, 0.44, 0.46, 0.48, 0.6, 0.62, 0.64, 0.66, 0.68, 0.7, 0.72, 0.74, 0.76, 0.78, 0.5, 0.52, 0.54, 0.56, 0.58, 0.8, 0.82, 0.84, 0.86, 0.88, 0.9, 0.92, 0.94, 0.96, 0.98, 0.99, 1.00, 1.10, 1.20, 1.30, 1.40, or 1.49 ppm.

It is a great advantage that a content of DO according to the invention is suitable for stabilizing the vitamin A and the further vitamins that can be comprised by the lipid emulsion of the medical product of the invention since such DO concentrations can be easily established without complicated technical instrumentation or manipulation of the solution.

Furthermore, it was an unexpected finding that a pH of 5-9 would be especially suitable to support stability of vitamin A in the lipid emulsion of the medical product of the invention and so far, pH had not been examined as a major parameter that may influence vitamin A stability. In particular, the fact that especially a pH in the range of 5.2-6.2, and especially a pH in the range of 5.7 to 6.1 is suitable to support long-term stability of vitamin A was surprising.

In embodiments, the pH can be any value in the claimed range. In embodiments, the pH of the lipid emulsion can be about 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9 or 9.0.

In embodiments of the invention, the vitamin A is provided in form of retinyl palmitate, retinyl acetate, retinal, retinol and/or retinoic acid, preferably retinyl palmitate.

In embodiments, the vitamin A of the medical product is predominantly present as retinyl palmitate. In embodiments, retinyl palmitate is the only form of vitamin A that is added to the lipid emulsion of the invention during the method of preparing it. In embodiments, the vitamin A of the lipid emulsion of the medical product of the invention consists of retinyl palmitate.

It was found that retinyl palmitate is particularly stable and ensures longer stability of vitamin A in a lipid emulsion of the medical product of the invention, as compared to certain other forms of vitamin A.

In further embodiments of the invention, the lipid emulsion comprises as a lipid component a low peroxide level oil, preferably selected from the group consisting of soybean oil with low peroxides, olive oil, medium chain triglycerides, fish oil, fish oil extract, krill oil, algae oil, safflower oil, sunflower oil, corn oil, coconut oil, palm kernel oil, rapeseed oil, fungal oil and hydrogenated oils or mixtures thereof.

A skilled person could not assume that the selection of a specific lipid components in the context of the lipid emulsion of the invention would be an important factor in enhancing stability of vitamin A and other vitamins that are comprised by the lipid emulsion. However, in the process of developing the product of the invention it was found that oils or lipids with low amounts of primary and secondary oxidation products are advantageous and may contribute to the stability of vitamin in a product according to the invention. For example, in case soybean oil is used, it should be ensured that comprises a low level of peroxides.

Accordingly, in embodiment the lipid emulsion of the medical product comprises as a lipid component an oil with a peroxide value of no more than 5 milliequivalents (mEq) O₂/kg, preferably no more than 3 mEq O₂/kg, more preferably no more than 1.5 mEq O₂/kg.

In embodiments of the invention, the lipid emulsion has a lipid concentration of 2-40 %, preferably 5-30 %, more preferably 5-25 %.

In embodiments, the lipid concentration of the invention can be any of about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 %.

In embodiments of the invention, the lipid emulsion may comprise an antioxidant agent, preferably selected from the group consisting of EDTA, tocopherol, ascorbyl palmitate and butylhydroxytoluen (BHT).

The use of an antixodant is advantageous and indicated in cases where lipid components and oils are used that are prone to oxidation, such as soybean oil. Such antioxidants prevent or slow down autooxidation of oils and lipids/fats by giving their hydrogen to free radicals formed in the initiation and propagation stages of autoxidation. There are various kinds of antioxidants, including natural antioxidants that can be extracted from parts of olive plants, green tea, sesame, medicinal plants, etc.

In embodiments of the invention, the medicinal product comprises 2000 - 4000 International Units (IU) vitamin A. This embodiment is preferable for provision of vitamin A to adult patients.

In embodiments, the medicinal product comprises 500 - 1500 International Units (IU) vitamin A and is preferably for us in neonate (neonates) and pre-term (pre-terms) babies. Preferably, a medical product of the invention for neo-nates comprises 500 - 1000 IU Vitamin A. Furthermore, a medical product of the invention for pre-terms preferably comprises 700 - 1500 IU vitamin A.

Accordingly, depending on the intended application the medical product of the invention can comprise any value of vitamin A in the range of 500 - 4000 IU, including about 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950, 2000, 2050, 2100, 2150, 2200, 1250, 2300, 2350, 2400, 2450, 2500, 2550, 2600, 2650, 2700, 2750, 2800, 2850, 2900, 2950, 3000, 3050, 3100, 3150, 3200, 3250, 3300, 3350, 3400, 3450, 3500, 3550, 3600, 3650, 3700, 3750, 3800, 3850, 3900, 3950 or 4000 IU.

In embodiments, the volume of the lipid emulsion comprising vitamin A and optionally vitamin D, E and/or K is in the range of 1 to 1000 ml. Any volume of the lipid emulsion of the invention within this range can be used, such as 2, 3, 4, 5, 6, 7, 8, 9, 10, 12, 14, 16, 18, 20, 24, 28, 32, 36, 40, 45, 50, 55, 60, 65, 70, 75, 80, 85, 90, 95, 100, 105, 110, 115, 120, 125, 130, 135, 140, 150, 160, 170, 180, 190, 200, 220, 240, 260, 280, 300, 320, 340, 360, 380, 400, 420, 440, 460, 480, 500, 520, 540, 560, 580, 600, 620, 640, 660, 680, 700, 720, 740, 760, 780, 800, 820, 840, 860, 880, 900, 920, 940, 960, 980, 1000 ml.

In embodiments of the invention, the lipid emulsion contains vitamin A and optionally also vitamin D, E and/or K and is comprised by the lipid formulation serving as the main lipid source of the invention. In such embodiments, the volume of the lipid emulsion comprising vitamin A and further vitamins is preferably in the range of about 50-1000 mL, preferably 101 mL-500 mL.

In further embodiments, the lipid emulsion comprising vitamins A and optionally also vitamins D, E, and/or K is an emulsion that is different from a major lipid source of the medical product of the invention. In embodiments, the medical product may comprise two lipid emulsions, wherein a first lipid emulsion is the emulsion comprising vitamin A and further vitamins provided in a smaller volume/chamber of a medical product of the invention, while a second emulsion serves as major lipid source and can be provided in a larger volume/chamber of a medical product of the invention, for example in the context of a ready-to-use TPN product, preferably in a separated chamber of the flexible container of the product of the invention. In such embodiments the vitamin A emulsion preferably has a volume in the range of about 1 to 150, more preferably about 1 to 100 ml, while the second lipid emulsion/chamber preferably has a volume of about 40-1000 ml, more preferably 50 - 500 ml. For example, the chamber comprising a first lipid emulsion comprising vitamin A as claimed herein may have a volume of 10 - 50 ml, such as 15 ml, 20 ml or 25 ml, while the second lipid emulsion may have a volume in the range of 100 to 500 ml, preferably about 200 ml.

As described above, in embodiments the lipid concentration of the lipid emulsion of the invention comprising vitamin A is in the range of 2-40%. Preferably, for further increased stability, it should be in the range of from 5 to 20% and preferably in the range of from 5 to 14%. Accordingly, in view of the possible volumes of the lipid emulsion, the lipid content (in gram) of the lipid solution can be calculated by any person skilled in the art. Furthermore, corresponding vitamin concentrations can be calculated. This is illustrated in the exemplary embodiments depicted in *Tables 1 and 2.*

**Table 1. Embodiments of the invention, wherein the lipid emulsion comprising vitamin A as claimed herein is comprised by a lipid emulsion serving as major lipid source.**

| | **Vitamin A in Lipid chamber** | | | | | | | |
|---|---|---|---|---|---|---|---|---|
| Vitamin A content IU/chamber | 2000 | 2000 | 2000 | 2000 | 4000 | 4000 | 4000 | 4000 |
| Bag size (mL) | 500 | 500 | 50 | 50 | 500 | 500 | 50 | 50 |
| Lipid concentration g/L | 150 | 200 | 150 | 200 | 150 | 200 | 150 | 200 |
| Lipid content g/chamber | 75 | 100 | 7,5 | 10 | 75 | 100 | 7,5 | 10 |
| Vitamin A concentration IU/g lipid | 26,66 | 20 | 266,66 | 200 | 53,33 | 40 | 533,33 | 400 |

**Table 2. Embodiments of the invention, wherein the lipid emulsion comprising vitamin A as claimed herein is provided in a smaller chamber that is different from the lipid chamber of the medical product.**

| | **Vitamin A in small chamber** | | | | | | | |
|---|---|---|---|---|---|---|---|---|
| Vitamin A content IU/chamber | 2000 | 2000 | 2000 | 2000 | 4000 | 4000 | 4000 | 4000 |
| Bag size (mL) | 1 | 1 | 100 | 100 | 1 | 1 | 100 | 100 |
| Lipid concentration g/L | 50 | 200 | 50 | 200 | 50 | 200 | 50 | 200 |
| Lipid content g/chamber | 0,05 | 0,2 | 5 | 20 | 0,05 | 0,2 | 5 | 20 |
| Vitamin A concentration IU/g lipid | 40.000 | 10.000 | 400 | 100 | 80.000 | 20.000 | 800 | 200 |

Accordingly, in embodiments of the invention, vitamin A can be present in the emulsion of the medical product at a concentration in the range of 20 to 80.000 IU vitamin A per gram of lipid (IU/gram).

In embodiments, the vitamin A concentration in the lipid emulsion can be any of about 20, 25, 30, 35, 40, 45, 50, 60, 70, 80, 90, 100, 110, 120, 130, 140, 150, 160, 170, 180, 190, 200, 220, 240, 260, 280, 300, 320, 340, 360, 380, 400, 420, 440, 460, 480, 500, 550, 600, 650, 700, 750, 800, 850, 900, 950, 1000, 1100, 1200, 1300, 1400, 1500, 1600, 1700, 1800, 1900, 2000, 2200, 2400, 2600, 2800, 3000, 3500, 4000, 4500, 5000, 5500, 6000, 6500, 7000, 7500, 8000, 8500, 9000, 9500, 10000, 11000, 12000, 13000, 14000, 15000, 16000, 17000, 180000, 19000, 20000, 21000, 22000, 23000, 24000, 25000, 26000, 27000, 280000, 29000, 30000, 31000, 32000, 33000, 34000, 35000, 36000, 37000, 380000, 39000, 40000, 41000, 42000, 43000, 44000, 45000, 46000, 47000, 480000, 49000, 50000, 51000, 52000, 53000, 54000, 55000, 56000, 57000, 580000, 59000, 60000, 61000, 62000, 63000, 64000, 65000, 66000, 67000, 680000, 69000, 70000, 71000, 72000, 73000, 74000, 75000, 76000, 77000, 780000, 79000, 80000 IU/gram lipid.

In embodiments of the medical product of the present invention, the lipid emulsion:
a. has a volume of 100 mL or less and vitamin A is present in the emulsion at a concentration of 1000 to 4000 IU/gram, preferably at a concentration of 1200 to 3000 IU/gram, or more preferably of about 2200 IU/gram of lipid; or
b. has a volume of more than 100 mL and vitamin A is present in the emulsion at a concentration of 20 to 300 IU/gram, more preferably at a concentration of about 40 to 180 IU/gram, especially preferably at a concentration of about 80 to 120 IU/gram.

In a further embodiment of the medical product of the invention, the lipid emulsion further comprises vitamins selected from the group consisting of B vitamins.

In embodiments, the lipid emulsion further comprises vitamin B2. In embodiments, the lipid emulsion further comprises vitamin B3. In embodiments, the lipid emulsion further comprises vitamin B5. In embodiments, the lipid emulsion further comprises vitamin B8. In embodiments, the lipid emulsion further comprises vitamin B9.

In embodiments, the lipid emulsion further comprises vitamin B2 and B3. In embodiments, the lipid emulsion further comprises vitamin B2 and B5. In embodiments, the lipid emulsion further comprises vitamin B2 and B8. In embodiments, the lipid emulsion further comprises vitamin B2 and B9. In embodiments, the lipid emulsion further comprises vitamin B2.

In embodiments, the lipid emulsion further comprises vitamin B3 and B5. In embodiments, the lipid emulsion further comprises vitamin B3 and B8. In embodiments, the lipid emulsion further comprises vitamin B3 and B9. In embodiments, the lipid emulsion further comprises vitamin B3.

In embodiments, the lipid emulsion further comprises vitamin B5 and B8. In embodiments, the lipid emulsion further comprises vitamin B5 and B9. In embodiments, the lipid emulsion further comprises vitamin B5.

In embodiments, the lipid emulsion further comprises vitamin B8 and B9. In embodiments, the lipid emulsion further comprises vitamin B8 and B12. In embodiments, the lipid emulsion further comprises vitamin B9.

In embodiments, the lipid emulsion further comprises vitamin B2, B3 and B5. In embodiments, the lipid emulsion further comprises vitamin B2, B3 and B8. In embodiments, the lipid emulsion further comprises vitamin B2, B3 and B9. In embodiments, the lipid emulsion further comprises vitamin B2 and B3.

In embodiments, the lipid emulsion further comprises vitamin B2, B5 and B8. In embodiments, the lipid emulsion further comprises vitamin B2, B5 and B9. In embodiments, the lipid emulsion further comprises vitamin B2 and B5.

In embodiments, the lipid emulsion further comprises vitamin B2, B8 and B9. In embodiments, the lipid emulsion further comprises vitamin B2, B8 and B12. In embodiments, the lipid emulsion further comprises vitamin B2 and B9.

In embodiments, the lipid emulsion further comprises vitamin B3, B5 and B8. In embodiments, the lipid emulsion further comprises vitamin B3, B5 and B9. In embodiments, the lipid emulsion further comprises vitamin B3 and B5.

In embodiments, the lipid emulsion further comprises vitamin B3, B8 and B9. In embodiments, the lipid emulsion further comprises vitamin B3 and B8. In embodiments, the lipid emulsion further comprises vitamin B3 and B9.

In embodiments, the lipid emulsion further comprises vitamin B5, B8 and B9. In embodiments, the lipid emulsion further comprises vitamin B5 and B8. In embodiments, the lipid emulsion further comprises vitamin B5 and B9.

In embodiments, the medical product of the invention comprises in the lipid phase of the lipid emulsion vitamin A, D, E and K. Furthermore, the medical product can comprise further vitamins, either in the lipid emulsion or in a different liquid provided in a different chamber of the flexible container. For example, vitamins B2, B3, B5, B8 and/or B9 can be comprised in the aqueous phase of the lipid emulsion or in a different solution of the product. Examples of preferable amounts of the respective vitamins that can be comprised by the medical product are listed in Table 3.

**Table 3. Suitable amounts and ranges of amounts of vitamins that can be used in the context of the invention. Indicated ranges are based on different ranges recommended in the art. The amounts refer to a medical product of the invention that provides one daily dose for one adult patient. The indicated ranges include the noted end-values. Ranges comprising any combination of disclosed end-values are considered as embodiments of the invention.**

| **Vitamin** | **Expressed as** | **Preferred amount #1** | **Preferred amount #2** | **ASPEN* 2004 and 2012** | **AuSPEN* 2016** |
|---|---|---|---|---|---|
| **Vitamin A (IU)** | Retinol | **3300** | 3500 | 3300 | 3500 |
| **Vitamin D3 (IU)** | Cholecalciferol or Ergochalciferol | **200** | 220 | 200 | 200 |
| **Vitamin E** | α-tocopherol | **10 mg** | 11.2 IU = 10.2 mg | 10 IU | 10 mg |
| **Vitamin K (µg)** | Phytomenadione | **150** | 0 | 150 | Not required |
| **Vitamin C (mg)** | Ascorbic acid | **200** | 125 | 200 | 110-150 |
| **Vitamin B1 (mg)** | Thiamin Chloride | **6** | 3.51 | 6 | 3 |
| **Vitamin B2 (mg)** | Riboflavine | **3.6** | 4.14 | 3.6 | 4-5 |
| **Vitamin B6 (mg)** | Puridoxine | **6** | 4.53 | 6 | 3 |
| **Vitamin B9 (pg)** | Folic Acid | **600** | 414 | 600 | 400 |
| **Vitamin B5 (mg)** | Panthothenic acid | **15** | 17.25 | 15 | 16-17 |
| **Vitamin B8 (pg)** | Biotin | **60** | 69 | 60 | 60 |
| **Vitamin B3/PP (mg)** | Nicotinamide | **40** | 46 | 40 | 40-47 |

| | | | | | |
|---|---|---|---|---|---|
| **ASPEN is the American Society for Parenteral and Enteral Nutrition and the indicated amounts are listed in the guidelines published in 2004 and 2012. AuSPEN is the Australasian society for parenteral and enteral nutrition and the indicated amounts are listed in the guidelines published 2016.* | | | | | |

In embodiments, the medicinal product comprises a light-protective outer wrapping. This is highly advantageous since vitamin A is light sensitive and light protection enhances its stability in the medical product.

In embodiments of the medical product, the flexible container comprises an oxygen barrier film, preferably with an oxygen permeability of less than 50 cc/m²/day. In further embodiments, the flexible container comprises an inner lining containing a polyolefin, wherein the inner lining is free of polyvinyl chloride (PVC), plasticizers, adhesives or latex. In embodiments, the flexible container comprises an oxygen barrier film, preferably with an oxygen permeability of less than 50 cc/m²/day, and an inner lining containing a polyolefin, wherein the inner lining is free of polyvinyl chloride (PVC), plasticizers, adhesives or latex.

In embodiments of the invention, the vitamin A is stable in the lipid emulsion for at least three months when stored at a temperature between 1 and 50 °C.

In embodiments of the invention, the vitamin A is stable in the lipid emulsion for at least 6 months, preferably 12 months, more preferably 18 months, most preferably 24 months at a temperature of up to 30°C, such as 25°C. For example, the vitamin A containing solution according to the invention is stable for 24 months at a temperature of 25°C. In embodiments, the vitamin A containing solution according to the invention is stable for 24 months at a temperature of 30°C.

It was surprising that the combination of features of the medical product of the invention, in particular a pH in the range of 5-9 and a DO concentration of no more than 1.5 ppm can ensure prolonged stability of vitamins A, D, E and K in the lipid phase of the lipid emulsion in the medical product, especially where terminally heat-sterilized products having a higher volume and/or MCBs comprising lipid emulsion formulations are concerned. The combination of the present features allows, for the first time, to stably introduce vitamin A into such medical products, specifically those of a higher volume as defined before, and especially MCBs providing for all macronutrients including lipid emulsions for parenteral nutrition, which require a higher heat exposure during terminal heat sterilization due to their size and high total (reconstituted) volume.

In embodiments, the lipid emulsion as formulated in the context of the present invention stabilizes vitamins A, optionally together with D, E and K, and potentially also in combination with one or more of the B vitamins listed above, which are preferably dissolved in the aqueous phase on the lipid emulsion for at least three months when stored at up to 40 °C.

In further embodiments of the invention, vitamin A, optionally in combination with vitamins D, E and K, is stable in the lipid emulsion for at least 6 months, preferably for at least 12 months, more preferably for at least 18 months, most preferably for at least 24 months.

In further embodiments of the invention, vitamin A, optionally in combination with vitamins D, E and K, is stable in the lipid emulsion for least 6 months, preferably 12 months, more preferably 18 months, most preferably 24 months, at a temperature of up to 25°C, or of up to 30 °C.

In yet further embodiments of the invention, vitamin A, optionally in combination with vitamins D, E and K, is stable in the lipid emulsion for least 6 months, preferably for at least 12 months, more preferably for at least 18 months, most preferably for at least 24 months, at a temperature of from about 1°C to 25°C.

In still further embodiments of the invention, vitamin A, optionally in combination with vitamins D, E and K, is stable in the lipid emulsion for at least 6 months, preferably for at least 12 months, more preferably for at least 18 months, most preferably for at least 24 months, at a regular storage temperature, including for example, but without limitation thereto, of temperatures of room temperature, varying from 15-30 °C, more preferably 18-25 °C, or storage in refrigerated conditions, such as from 1 to 10 °C, preferably 2 to 8, or 3 to 7 °C.

In embodiments of the invention, the lipid emulsion comprising vitamin A is a sterilized lipid emulsion. In some embodiments, the lipid emulsion of the medical product of the invention may be sterile.

The term "stable" as used herein in connection with vitamin A means that at least 50%, at least 60%, at least 70% or at least 80% of the amount of vitamin A initially provided in the product is still available after terminal heat-sterilization and storage of the terminally heat-sterilized product of the invention for at least 6 months at a temperature of from 1°C to 40°C and/or for at least 24 months at temperatures of from 1°C to 25°C. Preferably, at least 75%, at least 80%, at least 85%, at least 90% or at least 95% of the vitamin A provided in the product at the time it is produced is still available after terminal heat-sterilization and storage of the terminally heat-sterilized product of the invention for at least 6 months at a temperature of from 1°C to 40°C and/or for 24 months at a temperature from 1°C to 25°C. Generally, it will be assumed that the amount of vitamin A "provided in the product" corresponds to the amount stated in a product description. As mentioned before, the term "vitamin A", if not indicated otherwise, refers to cyanocobalamin and hydroxocobalamin.

It is an important advantage that the lipid emulsion of the present invention can be sterilized after preparation and packaging into a flexible bag, specifically said flexible bag is a multi-chamber container such as used for parental nutrition, which is preferably prepared from an oxygen-impermeable material, such as an oxygen barrier film, that may be sealed airtight and liquid tight without a significant loss of vitamin A and no loss of vitamin D, E and K, if present, and potentially further vitamins provided therein.

In certain preferred embodiments, the lipid emulsion of the invention undergoes terminal heat sterilization after preparation and filling of the lipid emulsion into the flexible container. Sterilization may occur prior to or after filling of the lipid emulsion into the flexible bag of the medical product of the invention, wherein terminal sterilization, specifically heat sterilization of the filled and sealed flexible container is clearly preferable in the context of the present invention.

Sterilization of the product according to the invention is preferably done by a terminal heat sterilization process but using gamma irradiation or any other sterilization technique may also be possible. Preferably, the medical product, specifically including any multi-chamber bag comprising the formulation according to the invention is terminally heat sterilized.

In embodiments, the vitamin A formulation according to the invention forms part of a terminally heat-sterilized multi-chamber container for parenteral nutrition and comprises at least two, at least three, at least four or at least five chambers, one of which accommodates the vitamin A formulation according to the invention.

In embodiments of the invention, the lipid emulsion does not comprise macronutrients selected from the group consisting of carbohydrates and proteins, and preferably the lipid emulsion does not comprise any nutrients besides lipids and vitamins. In embodiments, the aqueous phase of the lipid emulsion comprises water, which may not be considered a nutrient.

In embodiments, the lipid emulsion does not comprise carbohydrates. In embodiments, it does not comprise proteins. In further embodiments the lipid emulsion does not comprise proteins or amino acids. In embodiments, the lipid emulsion does not comprise electrolytes. In embodiments, the solution does not comprise trace elements. In embodiments, the emulsion only contains lipids and vitamins. In embodiments the lipid emulsion contains vitamins A, D, E and K as the only vitamins. In embodiments, the lipid emulsion contains vitamin A as only vitamin. In embodiments, the lipid emulsion contains vitamin A and D as the only vitamins. In embodiments, the lipid emulsion contains vitamin A and E as the only vitamins. In embodiments, the lipid emulsion contains vitamin A and K as the only vitamins. In embodiments, the lipid emulsion contains vitamin A, E and D as the only vitamins. In embodiments, the lipid emulsion contains vitamin A, K and D as the only vitamins. In embodiments, the lipid emulsion contains vitamin A, E and K as the only vitamins.

In embodiments of the invention, the emulsion and medical product are configured for parenteral administration. In further embodiments, the emulsion and medical product are configured for oral administration. In embodiments, the emulsion and medical product are configured for enteral administration.

In embodiments of the invention, the lipid emulsion is contained in one chamber of a multi chamber container having at least two, at least three, at least four, at least five or at least six chambers.

In further embodiments, the container comprises at least a first chamber containing a carbohydrate formulation, a second chamber containing an amino acid formulation, a third chamber containing a lipid formulation, and optionally comprises electrolytes and/or vitamins and/or trace elements in the first, second and/or third chamber.

In a further specified embodiment, the lipid emulsion is present in a fourth chamber.

In a specific embodiment, the lipid emulsion is present in a chamber comprising the lipid formulation serving as a macronutrient component of the medical product. As used herein, a lipid formulation serving as a macronutrient component relates to a liquid comprising lipids that is intended to cover the consumer's/patient's need for lipid in the context of, for example, TPN. The lipid emulsion of the invention comprising vitamins A, D, E and K can be comprised by this lipid formulation, which is usually provided in a large chamber of the medical product, and preferably has a volume in the range of 50 - 1000 ml, depending on the application. In alternative embodiments, the lipid emulsion of the invention comprising vitamins A, D, E and K is provided in a chamber that is separate from the chamber containing the lipid formulation serving as a macronutrient component of the medical product. In other words, the lipid emulsion can be either part of the lipid formulation or can be a separate solution that is present in a different chamber than the chamber of the lipid formulation.

In further embodiments, the container comprises at least a first chamber containing a carbohydrate formulation, a second chamber containing an amino acid formulation, a third chamber containing a lipid formulation, and optionally comprises electrolytes and/or vitamins and/or trace elements in the first, second, third chamber and/or forth chamber.

In embodiments, the container comprises at least a first chamber containing a carbohydrate formulation, a second chamber containing an amino acid formulation, a third chamber containing a lipid formulation, a fourth chamber containing trace elements (preferably in an aqueous solution), and optionally comprises electrolytes and/or vitamins in the first, second, third chamber and/or forth chamber.

In embodiments, the container comprises at least a first chamber containing a carbohydrate formulation, a second chamber containing an amino acid formulation, a third chamber containing a lipid formulation, a fourth chamber containing trace elements, optionally comprises electrolytes and/or vitamins in at least the first, second, third and/or a fourth chamber, and wherein at least one chamber comprises the lipid emulsion. Preferably, the container does comprise electrolytes and/or vitamins and/or trace elements in at least the first, second, third and/or fourth chamber. In certain of such embodiments, at least one chamber comprises the lipid emulsion, wherein the lipid emulsion is preferably either comprised by the lipid formulation of the third chamber or is present in a fifth chamber.

In embodiments, the container comprises at least a first chamber containing a carbohydrate formulation, a second chamber containing an amino acid formulation, a third chamber containing a lipid formulation, comprises electrolytes, trace elements and/or vitamins in a fourth and/or fifth chamber, and comprises the lipid emulsion in a further chamber.

In embodiments, the lipid emulsion comprises in the aqueous phase additional water-soluble vitamins, in particular one or more of the B vitamins disclosed herein, such as preferably vitamins B2, B5 and/or B12.

In embodiments, the lipid emulsion does not contain vitamin B1 and vitamin C.

In embodiments, the container comprises at least a first chamber containing a carbohydrate formulation, a second chamber containing an amino acid formulation, a third chamber containing a lipid formulation, a fourth chamber containing trace elements, and a fifth chamber containing the lipid emulsion.

In embodiments of the invention, the lipid emulsion is contained in one chamber of a multi chamber container having at least two, at least three, at least four, at least five or at least six chambers. In embodiments of the invention, the container is made from an oxygen-impermeable, flexible material. Such a container can for example comprise an oxygen barrier film, preferably with an oxygen permeability of less than 50 cc/m²/day.

In a further aspect, the present invention relates to a method of preparing a medical product of the present invention for preventing or correcting vitamin A deficiency in a patient, wherein the lipid emulsion comprises a lipid phase and an aqueous phase and is prepared by the steps comprising
a. separately heating up the lipid phase and the aqueous phase to a temperature of from 60°C to 90°C under agitation;
b. adding vitamin A, and optionally vitamin D, vitamin E and/or vitamin K to the lipid phase;
c. preparing the pre-emulsion by transferring the lipid phase to the aqueous phase under agitation;
d. homogenizing the pre-emulsion at a temperature of from 40°C to 60°C under pressure;
e. optionally adding water to adjust the required volume and concentrations;
f. adjusting the lipid emulsion to a concentration of dissolved oxygen (DO) of no more than 1.5 ppm and a pH value of 5-9, and
g. sterilizing the solution, preferably by terminal heat sterilization, more preferably by terminal heat sterilization with moist heat.

In embodiments, in the step a. the lipid phase and the aqueous phase are heated up under agitation up to a temperature of 70°C - 80 °C.

In another aspect, the invention relates to a sterilized lipid emulsion for parenteral administration for use in preventing or correcting vitamin A deficiency, comprising (a) vitamin A, preferably in the form of retinyl palmitate, in a lipid phase of a lipid emulsion, (b) optionally vitamin D, vitamin E and/or vitamin K in the lipid phase of the lipid emulsion, (c) no more than 1.5 ppm DO, and (d) with a pH of 5-9.

Sterilizing of the lipid emulsion may occur prior or after filling the solution into a flexible container of the invention.

Furthermore, the invention relates to a method of preventing or correcting vitamin A deficiency in a patient, the method comprising administering a lipid emulsion comprising (a) vitamin A, preferably in the form of retinyl palmitate, in a lipid phase of a lipid emulsion, (b) optionally vitamin D, vitamin E and/or vitamin K in the lipid phase of the lipid emulsion, (c) no more than 1.5 ppm DO, and (d) with a pH of 5-9.

In embodiments of the method of preventing or correcting vitamin A deficiency in a patient according to the present invention, the method comprises administering the solution for maintaining plasma levels of vitamin A and preventing depletion of endogenous stores in a patient receiving total parenteral nutrition.

In the context of the method of preventing or correcting vitamin A deficiency in a patient, the patients are adult patients. In embodiments, the patients are pediatric patients, such as neonates, pre-terms, infants, children or adolescents.

As used herein, the term "adult" refers to persons of 20 years of age and older. The term "pediatric" refers to neonates, including premature (pre-terms), full term, and post-mature neonates of up to one month of age; infants of between one month and one year of age; children of between one and up to 12 years of age, and adolescents of between 13 and up to 19 years of age.

All features disclosed in the context of the medical product for preventing or correcting vitamin A deficiency in a patient of the invention also relate to the method of preparing such a medical product and are herewith disclosed also in the context of the method of preparing the medical product and the other way around. The same holds true for the sterile or sterilized lipid emulsion of the invention and the method of preventing or correcting vitamin A deficiency in a patient according to the present invention.

### DETAILED DESCRIPTION OF THE INVENTION

All cited documents of the patent and non-patent literature are hereby incorporated by reference in their entirety.

The present invention is directed to a preferably terminally heat-sterilized medical product for preventing or correcting vitamin A deficiency in a patient comprising a lipid emulsion in a flexible container, the lipid emulsion comprising (a) vitamin A, preferably in the form of retinyl palmitate, in the lipid phase of the lipid emulsion, (b) optionally additionally vitamin D, vitamin E and/or vitamin K in the lipid phase of the lipid emulsion, and (c) no more than 1.5 ppm dissolved oxygen (DO), (d) wherein the pH of the lipid emulsion is from 5 to 9.

As disclosed herein, the term medical product relates to any product intended to be used for medical purposes, preferably for clinical nutrition. The medical product of the invention is intended and designed for the medical purpose of preventing or correcting vitamin A deficiency in a patient.

The term vitamin A relates to a group of fat-soluble unsaturated nutritional organic compounds that includes retinol, retinal, and retinoic acid, which are the compounds that are active in the body. Furthermore, the term comprises several provitamin A carotenoids (most notably beta-carotene), including the storage forms of vitamin A.

Vitamin A has multiple functions: it is important for growth and development, for the maintenance of the immune system, and for good vision. Vitamin A is needed by the retina of the eye in the form of retinal, which combines with protein opsin to form rhodopsin, the light-absorbing molecule necessary for both low-light (scotopic vision) and color vision. Vitamin A also functions in a very different role as retinoic acid (an irreversibly oxidized form of retinol), which is an important hormone-like growth factor for epithelial and other cells.

In foods of animal origin, vitamin A is mostly present in form of esters, primarily retinyl palmitate, which is converted to retinol (chemically an alcohol) in the small intestine. The retinol form functions as a storage form of vitamin A and can be converted to and from its visually active aldehyde form, retinal.

All forms of vitamin A have a beta-ionone ring to which an isoprenoid chain is attached, called a retinyl group. Both structural features are essential for vitamin activity. The orange pigment of carrots (beta-carotene) can be represented as two connected retinyl groups, which are used in the body to contribute to vitamin A levels. Alpha-carotene and gamma-carotene also have a single retinyl group, which give them some vitamin activity. None of the other carotenes have vitamin activity. The carotenoid beta-cryptoxanthin possesses an ionone group and has vitamin activity in humans.

Vitamin A can be found in two principal forms in foods, retinol and carotenes. Retinol, the form of vitamin A absorbed when eating animal food sources, is a yellow, fat-soluble substance. Since the pure alcohol form is unstable, the vitamin is found in tissues in a form of retinyl ester. It is also commercially produced and administered as esters such as retinyl acetate or palmitate. The carotenes alpha-carotene, beta-carotene, gamma-carotene; and the xanthophyll beta-cryptoxanthin (all of which contain beta-ionone rings), but no other carotenoids, function as provitamin A in herbivores and omnivore animals, which possess the enzyme beta-carotene 15,15'-dioxygenase which cleaves beta-carotene in the intestinal mucosa and converts it to retinol.

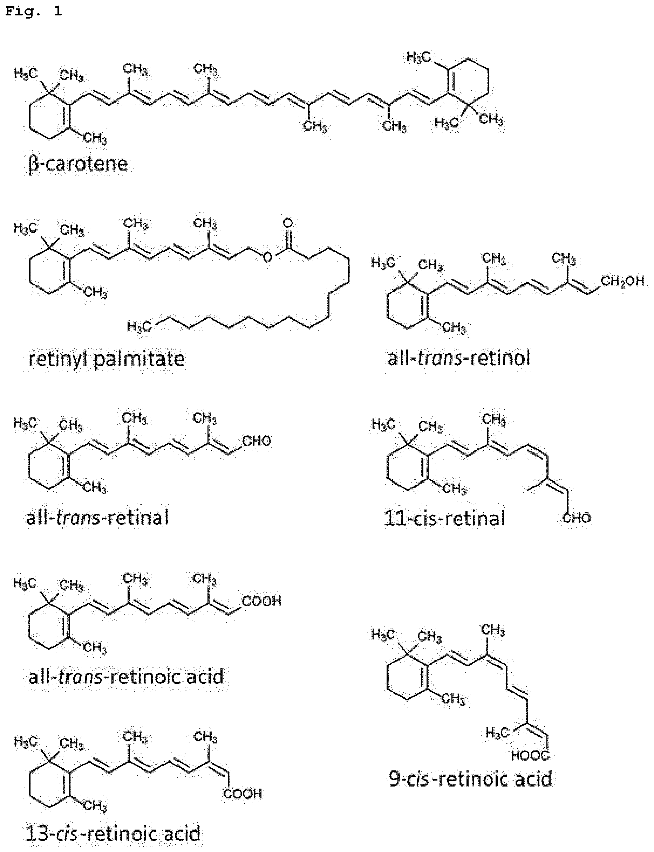

In the context of the invention, example of vitamin A include retinyl palmitate, retinyl acetate, retinal, retinol, retinoic acid, and/or the examples displayed in Figure 1.

In the context of the invention, the use of retinyl palmitate is particularly preferred, since it was found that this form of vitamin A is associated with a very good stability in the context of the medical product of the invention.

Retinyl palmitate, also referred to as vitamin A palmitate or retinol palmitate, is the ester of retinol and palmitic acid, with formula C₃₆H₆₀O₂.

Vitamin A deficiency refers to a condition of a patient who does not have enough vitamin A in his body. This can cause several health problems. The patient group of the invention comprises all patients or patient groups that are at risk of developing a vitamin A deficiency or have already developed a vitamin A deficiency. In particular, the medical product of the invention is intended to correct or treat a vitamin A deficiency that has already occurred in a patient. Furthermore, the invention may be used to prevent the occurrence of vitamin A deficiency in patients at risk of or suspected of developing a vitamin A deficiency, for example due to severely compromised intestinal function, total or partial parenteral nutrition, gastrointestinal bypass surgery, or advanced age. In the present context of the invention, prevention is considered to encompass both absolute prevention, i.e. stopping of a disease developing at all, and/or a preventive measure that lowers the risk or likelihood of the patient developing the unwanted medical conditions or slows or delays the development, first occurrence and/or progression of the disease.

Vitamin A deficiency is estimated to affect approximately one third of children under the age of five around the world. It is estimated to claim the lives of 670,000 children under five annually. Between 250,000 and 500,000 children in developing countries become blind each year owing to vitamin A deficiency, with the highest prevalence in Africa and southeast Asia. Vitamin A deficiency is "the leading cause of preventable childhood blindness", according to UNICEF. It also increases the risk of death from common childhood conditions such as diarrhea. UNICEF regards addressing vitamin A deficiency as critical to reducing child mortality, the fourth of the United Nations' Millennium Development Goals. Accordingly, the application of the medical product of the invention in the treatment of children, neonates, pre-terms and adolescence is an important aspect of the invention.

Vitamin A deficiency can occur as either a primary or a secondary deficiency. A primary vitamin A deficiency occurs among children and adults who do not consume an adequate intake of provitamin A carotenoids from fruits and vegetables or preformed vitamin A from animal and dairy products. Early weaning from breastmilk can also increase the risk of vitamin A deficiency.

Secondary vitamin A deficiency is associated with chronic malabsorption of lipids, impaired bile production and release, and chronic exposure to oxidants, such as cigarette smoke, and chronic alcoholism. Vitamin A is a fat-soluble vitamin and depends on micellar solubilization for dispersion into the small intestine, which results in poor use of vitamin A from low-fat diets. Zinc deficiency can also impair absorption, transport, and metabolism of vitamin A because it is essential for the synthesis of the vitamin A transport proteins and as the cofactor in conversion of retinol to retinal. In malnourished populations, common low intakes of vitamin A and zinc increase the severity of vitamin A deficiency and lead physiological signs and symptoms of deficiency. A study in Burkina Faso showed major reduction of malaria morbidity by use of combined vitamin A and zinc supplementation in young children. The invention relates to all kinds of vitamin A deficiencies, including primary and secondary deficiency.

Due to the unique function of retinal as a visual chromophore, one of the earliest and specific manifestations of vitamin A deficiency is impaired vision, particularly in reduced light - night blindness. Persistent deficiency gives rise to a series of changes, the most devastating of which occur in the eyes. Some other ocular changes are referred to as xerophthalmia.

Adequate supply, but not excess vitamin A, is especially important for pregnant and breastfeeding women for normal fetal development and in breastmilk. Deficiencies cannot be compensated by postnatal supplementation. Excess vitamin A, which is most common with high-dose vitamin supplements, can cause birth defects and therefore should not exceed recommended daily values.

The medical product of the invention can be used in the treatment of patients having, developing or being at risk of developing vitamin A deficiency. The treatment can aim to prevent or correct a vitamin A deficiency in this patient group.

In the context of the invention, the medical product comprises a flexible container or flexible bag, preferably made from an oxygen-impermeable material. In the context of the invention, an example of an oxygen-impermeable material is an oxygen barrier film, preferably with an oxygen permeability of less than 50 cc/m²/day.

The term "dissolved oxygen" (DO) refers to the level of free, non-compound oxygen present in liquids, such as water, lipid emulsions or other liquids or solutions. Oxygen saturation (symbol SO₂) is a relative measure of the concentration of oxygen that is dissolved or carried in a given medium as a proportion of the maximal concentration that can be dissolved in that medium. It can be measured with a dissolved oxygen probe such as an oxygen sensor or an optode in liquid media, usually water.

Dissolved oxygen is usually reported in milligrams per liter (mg/L) or as a percent of air saturation. However, studies also report DO in parts per million (ppm) or in micromoles (µmol). 1 mg/L is equal to 1 ppm. The relationship between mg/L and % air saturation varies with temperature, pressure and salinity of the water. One micromole of oxygen is equal to 0.022391 milligrams. Thus 100 µmol/L O₂ is equal to 2.2 mg/L O₂. To calculate dissolved oxygen concentrations from air saturation, it is necessary to know the temperature and salinity of the sample. Barometric pressure has already been accounted for as the partial pressure of oxygen contributes to the percent air saturation. Salinity and temperature can then be used in Henry's Law to calculate what the DO concentration would be at 100% air saturation. However, it is easier to use an oxygen solubility chart. These charts show the dissolved oxygen concentration at 100% air saturation at varying temperatures, and salinities. This value can then be multiplied by the measured percent air saturation to calculate the dissolved oxygen concentration [Fondriest Environmental, Inc. "Dissolved Oxygen." Fundamentals of Environmental Measurements. 19 Nov. 2013.].

Oxygenation of liquids occurs for example through exposure of the liquids to gases containing oxygen. For example, exposure of a liquid sample to the atmosphere comprising around 21% O₂ leads to oxygenation through diffusion of the gaseous oxygen into the liquid. This process can be accelerated for example by stirring, flushing the liquid with an oxygen-containing gas or similar techniques known to the skilled person.

There are several methods available in the art for measuring dissolved oxygen concentrations. Modern techniques involve either an electrochemical or optical sensor, where the dissolved oxygen sensor is attached to a meter for spot sampling and laboratory applications or to a data logger, process monitor or transmitter for deployed measurements and process control. An example is the fiber-optic oxygen meter Microx TX3 from PreSens Precision Sensing GmbH (Germany) for gaseous and dissolved O₂. The colorimetric method offers a basic approximation of dissolved oxygen concentrations in a sample. There are two methods designed for high-range and low-range dissolved oxygen concentrations. These methods are quick and inexpensive for basic projects but limited in scope and subject to error due to other redoxing agents that may be present in the water. The traditional method is the Winkler titration.

Methods of deoxygenation are known to a person skilled in the art, including for example gas-flushing with suitable gases, such as nitrogen, or by use of vacuum deaerators.

In embodiments of the invention, the lipid emulsion of the medical product is a sterilized emulsion. In the context of the invention, the term "sterilized" relates to a solution or emulsion that has undergone a process of sterilization. Sterilization refers to any process that eliminates, removes, kills, or deactivates all forms of life (in particular referring to microorganisms such as fungi, bacteria, viruses, spores, unicellular eukaryotic organisms such as Plasmodium, etc.) and other biological agents like prions present in a specific surface, object or fluid, for example food or biological culture media. Sterilization can be achieved through various means, including heat, chemicals, irradiation, high pressure, and filtration. Sterilization is distinct from disinfection, sanitization, and pasteurization, in that those methods reduce rather than eliminate all forms of life and biological agents present. After sterilization, an object is referred to as being sterile or aseptic.

According to one embodiment of the invention, sterilization is done by heat. According to another embodiment of the invention, sterilization involves heating under pressure in the presence of water to generate steam; this method is recommended by various pharmacopeias and regulatory authorities. Generally, said steam sterilization is performed in an autoclave and can be used for drug products, medical devices, plastic bags and other single-use equipment, glass containers, surgical dressings and more.

Other methods encompass sterilization with moist heat. The tern "moist heat" as used herein includes the use of saturated steam, steam air and hot water cascade or water spray sterilization. According to one embodiment of the invention, sterilization with moist heat is preferable because it allows to reduce the total heat exposure of a solution to be sterilized.

Sterilization can also be achieved via dry heating. Much higher temperatures (180-200 °C) are required for this method. Dry heating is commonly used to sterilize glassware, metal and other surfaces.

In embodiments of the invention, sterilization of the lipid emulsion and/or the medical product of the invention is performed in a sterilization process with C₀ value of no more than 130 minutes, and preferably no more than 120, 110, 100, 90 or 80 minutes. In embodiments, the C₀ value is no more than 70, 60, 50 or 40 minutes. In embodiments, the F₀ value of the sterilization process is at least 6, preferably at least 8 minutes. In embodiments, the C₀ value of the sterilization process is in the range of 8 to 100, preferably 8 to 80 minutes. As used herein, the C₀ value can be understood as the time (in minutes) during which the vitamin B12 formulation is at a temperature of 100 °C or more during the sterilization process. In general, C₀ is a physical parameter used to quantify the total heat consumption of a sample.

In certain standard sterilization protocols, sterilization is done at 121 °C, which can be considered a standard temperature for sterilization. If a material is sterilized at a temperature other than 121 °C, the time of sterilization leading to the same result of killing microorganisms changes. Some materials are heat sensitive and cannot be sterilized at 121 °C. The so-called F₀ value is used to determine the exposure time of material for sterilization at a particular temperature. The F₀ value is the time in minutes for the specified temperature that gives the same thermal lethality as sterilization for 1 minute at 121 °C. In other words, the F₀ is defined as the number of equivalent minutes of steam sterilization at temperature 121.1 °C (250 °F) delivered to a container or unit of product calculated using a z-value of 10 °C.

Z-value is a term used in microbial thermal death time calculations. It is the number of degrees the temperature has to be increased to achieve a tenfold (i.e. 1 log10) reduction in the D-value. The D-value of an organism is the time required in a given medium, at a given temperature, for a ten-fold reduction in the number of organisms. It is useful when examining the effectiveness of thermal inactivations under different conditions, for example in food cooking and preservation. The z-value is a measure of the change of the D-value with varying temperature, and is a simplified version of an Arrhenius equation and it is equivalent to z=2.303 RT T_{ref}/E.

The z-value of an organism in a particular medium is the temperature change required for the D-value to change by a factor of ten, or put another way, the temperature required for the thermal destruction curve to move one log cycle. It is the reciprocal of the slope resulting from the plot of the logarithm of the D-value versus the temperature at which the D-value was obtained. While the D-value gives the time needed at a certain temperature to kill 90% of the organisms, the z-value relates the resistance of an organism to differing temperatures. The z-value allows calculation of the equivalency of two thermal processes, if the D-value and the z-value are known.

The F0 value can be calculated by the equation F0 = Δt x 10^(T-121/Z), wherein T = Temperature for Sterilization, Δt = lmin and Z = 10°C. For Example, if sterilization is carried out at 115 °C, the F0 value can be calculated as F0 = 1 x 10^(115-121/ 10) = 1 x 10^(-6/ 10) = 1 x 10^(-0.6) = 3.98 minutes. This means that the thermal lethality of 1 minute at 121 °C is equal to the lethality of 3.98 minutes at 115 °C.

Exposure to radiation is another sterilization method used throughout the industry. Gamma radiation is the most common, though other options include infrared and ultraviolet radiation and high-velocity electrons. Radiation is typically used for the sterilization of single-use components/systems, but it can also be used for packaged drug products.

Treatment with gases is also a sterilization alternative. Such gases include ethylene oxide, formaldehyde, glutaraldehyde, propylene oxide, hydrogen peroxide and chlorine dioxide. This method is more commonly used to sterilize clean-room suites. Sterilization via filtration is the only option if the other processes are not suitable for the specific product or component. In filtration, the final drug product solution is passed through a filter that has been produced under aseptic manufacturing conditions and designed with appropriate pore sizes/surface chemistries that remove bacteria via size exclusion, entrapment, electrostatic attraction and other modalities.

As used herein, a nutrient is a substance used by an organism, such as a human, to survive, grow, and reproduce. Nutrients can be incorporated into cells for metabolic purposes or excreted by cells to create non-cellular structures, such as hair, scales, feathers, or exoskeletons. Some nutrients can be metabolically converted to smaller molecules in the process of releasing energy, such as for carbohydrates, lipids, proteins/amino acids, and fermentation products (ethanol or vinegar), leading to end-products of water and carbon dioxide. All organisms require water. Essential nutrients for animals and humans are the energy sources, some of the amino acids that are combined to create proteins, a subset of fatty acids, vitamins and certain minerals/trace elements.

A classification used primarily to describe nutrient needs of animals divides nutrients into macronutrients and micronutrients. Consumed in relatively large amounts, macronutrients (carbohydrates, lipids/fats, proteins/amino acids, water) are used primarily to generate energy or to incorporate into tissues for growth and repair. Micronutrients are needed in smaller amounts; they have subtle biochemical and physiological roles in cellular processes, like vascular functions or nerve conduction. Inadequate amounts of essential nutrients, or diseases that interfere with absorption, result in a deficiency state that compromises growth, survival and reproduction.

Macronutrients comprise carbohydrates, proteins, lipids, and water. Macronutrients are defined as a class of chemical compounds which humans consume in the largest quantities and which provide humans with the bulk of energy. While water does make up a large proportion of the total mass ingested as part of a normal diet, it does not provide any nutritional value. Carbohydrates comprise glucose, sucrose, ribose, amylose (a major component of starch), amylopectin, maltose, galactose, fructose, lactose. Proteins are composed of amino acids comprising standard amino acids alanine, arginine, aspartic acid (aspartate), asparagine, cysteine, glutamic acid (glutamate), glutamine, glycine, histidine, isoleucine (branched chain amino acid), leucine (branched chain amino acid), lysine, methionine, phenylalanine, proline, serine, threonine, tryptophan, tyrosine, valine (branched chain amino acid). Lipids (or fats) comprise saturated fats such as butyric acid (C4), caproic acid (C6), caprylic acid (C8), capric acid (C10), lauric acid (C12), myristic acid (C14), pentadecanoic acid (C15), palmitic acid (C16), margaric acid (C17), stearic acid (C18), arachidic acid (C20), behenic acid (C22), lignoceric acid (C24), cerotic acid (C26); monounsaturated fats such as myristol, pentadecanoic, palmitoyl, heptadecenoic, oleic acid, eicosen, erucic acid, nervonic acid; polyunsaturated fats such as linoleic acid (LA) - an essential fatty acid, α-Linolenic acid (ALA) - an essential fatty acid, stearidonic acid (SDA), arachidonic acid (ETA), timnodonic acid (EPA), clupanodonic acid (DPA), cervonic acid (DHA); essential fatty acids, such as α-Linolenic acid ALA (18:3) Omega-3 fatty acid and Linoleic acid LA (18:2) Omega-6 fatty acid, being the starting point for other important omega-acids (e.g. DHA, EPA) .

Micronutrients are essential elements required by human in small quantities throughout life to orchestrate a range of physiological functions to maintain health, in particular vitamins and dietary minerals/trace elements. Trace elements/trace minerals/dietary minerals include Boron, Cobalt (as a component of vitamin B12), Fluoride, Chromium, Copper, Iodine, Iron, Manganese, Molybdenum, Selenium and Zinc.

In the context of the medical product of the invention, trace elements may be provided as chloride or sodium salts, such as zinc chloride, iron chloride, copper chloride, sodium selenite, sodium selenate, manganese chloride, sodium fluoride, potassium iodide, chromium chloride, sodium molybdate.

Vitamins comprise, for example, Vitamin B complex, Vitamin B1 (thiamin), Vitamin B2 (riboflavin), Vitamin B3 (niacin), Vitamin B5 (pantothenic acid), Vitamin B6 group (Pyridoxine, Pyridoxal-5-Phosphate, Pyridoxamine), Vitamin B7 (biotin), Vitamin B9 (folate), Vitamin B12 (cobalamin), Choline, Vitamin A (e.g. retinol (see also - provitamin A carotenoids)), Vitamin C (Ascorbic acid), Vitamin D (vitamin D2 (Ergocalciferol), vitamin D3 (Cholecalciferol)), Vitamin E (tocopherols and tocotrienols), Vitamin K (Vitamin K1 (phylloquinone), Vitamin K2 (menaquinone)).

The medical product of the present invention comprises a lipid emulsion comprising in its lipid phase the fat-soluble vitamin A and optionally also vitamins D, E and K.

As used herein, the term vitamin D relates to a group of fat-soluble secosteroids responsible for increasing intestinal absorption of calcium, magnesium, and phosphate, and multiple other biological effects. In humans, the most important compounds in this group are vitamin D3 (also known as cholecalciferol) and vitamin D2 (ergocalciferol).

The major natural source of vitamin D is synthesis of cholecalciferol in the lower layers of skin epidermis through a chemical reaction that is dependent on sun exposure (specifically UVB radiation). Cholecalciferol and ergocalciferol can be ingested from the diet and from supplements. Only a few foods, such as the flesh of fatty fish, naturally contain significant amounts of vitamin D.

In embodiments of the invention, vitamin D comprises or consists of vitamin D3. In embodiments of the invention, vitamin D comprises vitamin D5, D4, D3, D2 and/or D1.

Vitamin D1 is a 1:1 mixture of molecular compounds of ergocalciferol with lumisterol. Vitamin D2 is ergocalciferol made from ergosterol. Vitamin D3 is cholecalciferol, which is made from 7-dehydrocholesterol in the skin. Vitamin D4 is 22-dihydroergocalciferol. Vitamin D5 is sitocalciferol, which is made from 7-dehydrositosterol.

The medical product of the invention can be used, in embodiments, for preventing or correcting vitamin D deficiency in a patient. An estimated one billion people worldwide are either vitamin D insufficient or deficient. A diet with insufficient vitamin D in conjunction with inadequate sun exposure causes vitamin D deficiency. Severe vitamin D deficiency in children causes rickets, a softening and weakening of bones, which is a rare disease in the developed world. Vitamin D deficiency is found worldwide in the elderly and remains common in children and adults. Deficiency results in impaired bone mineralization and bone damage which leads to bone-softening diseases, including rickets in children and osteomalacia in adults. Low blood calcifediol can result from avoiding the sun. Being deficient in vitamin D can cause intestinal absorption of dietary calcium to fall to 15%. When not deficient, an individual usually absorbs between 60-80%.

As used herein, vitamin E is a group of eight fat soluble compounds that include four tocopherols and four tocotrienols. Both the tocopherols and tocotrienols occur in α (alpha), β (beta), γ (gamma) and δ (delta) forms, as determined by the number and position of methyl groups on the chromanol ring. All eight of these vitamers feature a chromane double ring, with a hydroxyl group that can donate a hydrogen atom to reduce free radicals, and a hydrophobic side chain which allows for penetration into biological membranes. Of the many different forms of vitamin E, gamma-tocopherol (γ-tocopherol) is the most common form found in the North American diet, but alpha-tocopherol (α-tocopherol) is the most biologically active. All forms of vitamin E can be comprised by the medical product of the invention.

Vitamin E deficiency, which is rare and usually due to an underlying problem with digesting dietary fat rather than from a diet low in vitamin E, can cause nerve problems. Vitamin E is a fat-soluble antioxidant protecting cell membranes from reactive oxygen species.

The medical product of the invention can be used, in embodiments, for preventing or correcting vitamin E deficiency in a patient. Worldwide, government organizations recommend adults consume in the range of 7 to 15 mg per day.

As used herein, the term vitamin K relates to a group of structurally similar, fat-soluble vitamins found in foods and in dietary supplements. The human body requires vitamin K for complete synthesis of certain proteins that are needed for blood coagulation or for controlling binding of calcium in bones and other tissues. The complete synthesis involves final modification of these so-called "gla-proteins" by the enzyme gamma-glutamyl carboxylase that uses vitamin K as a cofactor. This modification allows them to bind (chelate) calcium ions, which they cannot do otherwise.

Without vitamin K, blood coagulation is seriously impaired, and uncontrolled bleeding occurs. Preliminary clinical research indicates that deficiency of vitamin K may weaken bones, potentially leading to osteoporosis, and may promote calcification of arteries and other soft tissues. The medical product of the invention may be suitable and could be used for the treatment vitamin K deficiency in a patient.

Chemically, the vitamin K family comprises 2-methyl-1,4-naphthoquinone (3-) derivatives. Vitamin K includes two natural vitamers: vitamin K1 and vitamin K2. Vitamin K2, in turn, consists of a number of related chemical subtypes, with differing lengths of carbon side chains made of isoprenoid groups of atoms. In embodiments, all these forms of vitamin K can be comprised by the medical product and preferably the lipid phase of the lipid emulsion of the present invention.

Vitamin K1, also known as phylloquinone or phytomenadione, is made by plants, and is found in highest amounts in green leafy vegetables because it is directly involved in photosynthesis. It may be thought of as the plant form of vitamin K. It is active as a vitamin in animals and performs the classic functions of vitamin K, including its activity in the production of blood-clotting proteins. Animals may also convert it to vitamin K2 in the form of the homologue MK-4 (known as menaquinone).

Bacteria in the gut flora can also convert K1 into vitamin K2 as MK-4. In addition, bacteria typically lengthen the isoprenoid side chain of vitamin K2 to produce a range of vitamin K2 forms (vitamers), most notably the MK-7 to MK-11 homologues of vitamin K2. All forms of K2 other than MK-4 can only be produced by bacteria, which use these during anaerobic respiration. The MK-7 and other bacterially derived forms of vitamin K2 exhibit vitamin K activity in animals, but MK-7's extra utility over MK-4, if any, is unclear and is a matter of investigation.

Because a synthetic form of vitamin K, vitamin K3 (menadione), may be toxic by interfering with the function of glutathione, it is no longer used to treat vitamin K deficiency. Accordingly, embodiments of the medical product of the invention do not comprise vitamin K3.

Electrolytes, such as sodium, potassium, chloride, calcium, magnesium, phosphate and bicarbonate may also be classified as nutrients.

Besides the lipid emulsion comprising vitamin A and optionally also vitamins D, E and K, the medical product of the invention may comprise further solutions/liquids comprising nutrients, such as macronutrients and micronutrients. The liquids of the medical product may be reconstituted before administration to a patient. Administration of the one or more solutions of the product, potentially after reconstitution of the one or more solutions, can occur through various routes of administration, such as parenterally, orally or enterally.

As used herein, "reconstituted solution" refers to a solution for parenteral administration, which is generated by admixing the content of the chambers of a multi-chamber container before use.

Parenteral administration is preferred in the context of the invention. Parenteral nutrition (PN) is the feeding of specialist nutritional products to a person intravenously, bypassing the usual process of eating and digestion. It is called total parenteral nutrition (TPN) or total nutrient admixture (TNA) when no significant nutrition is obtained by other routes, and partial parenteral nutrition (PPN) when nutrition is also partially enteric. It may be called peripheral parenteral nutrition (PPN) when administered through vein access in a limb rather than through a central vein as central venous nutrition (CVN). Enteral food administration is via the human gastrointestinal tract and contrasts with parenteral administration.

In the context of the medical product of the invention, the lipid emulsion is provided in a flexible container. In embodiments of the invention, the lipid emulsion is contained in one chamber of a multi chamber container having at least two, at least three, at least four, at least five or at least six chambers.

As used herein, the term "flexible container" refers to a container or bag made of a flexible material, such as bags made from plastic films. The term does not encompass polymeric rigid or semirigid containers.

Flexible containers or bags of the invention can be made of materials comprising, without limitation, polyvinyl chloride (PVC), polypropylene (PP), polyethylene (PE), ethylene vinyl alcohol (EVOH), ethylene-vinyl acetate (EVA) and all possible copolymers, essentially any synthetic material suitable for containing the components to be administered.

Oxygen impermeable flexible containers are made of gas barrier films that block oxygen migration to the outside of the container. Different technologies have been developed in order to provide oxygen barrier to transparent films, such as PE films or polyethylene terephthalate films. The main technologies are the following: (1) Coating with high barrier materials, generally inorganic oxide layers (e.g. SiOx or Al₂O₃); (2) Multilayer films, wherein an inner layer consists a barrier material such as EVOH, polyamide, aluminum, halogenated polyvinylidene such as PVDC, amorphous nylon or crystalline nylon or combination of both, copolymers of ethylene vinyl alcohol copolymer layer (EVOH), polyolefins, including combinations of two or more of the above layers, and wherein the outer layers consist of structural polymer (e.g. PE, PP or PET).

The disclosure therefore also provides for a flexible container, preferably a multi-chamber container for parenteral or nutritional formulations which may be prepared from any of the beforementioned flexible films. For example, the container may be in the form of a bag having one or multiple compartments or chambers. The container, such as a bag, may include at least two chambers, but may also contain three, four, five or six or more chambers, and in one preferred embodiment, two or three chambers.

Suitable containers, including soft bags, typically are sterile, non-pyrogenic, single-use, and/or ready-to-use. The multi-chamber containers are particularly useful for holding a parenteral nutrition product for adults, children or neonates and can provide a carbohydrate formulation as disclosed herein in the first chamber, an amino acid formulation as disclosed herein in a second chamber, and a lipid formulation as disclosed herein in a third chamber of the container.

The multi-chamber container may include vertical chambers as disclosed in U.S. Patent Publication No. 2007/0092579. For example, the multi-chamber container may be configured as a bag that includes two, three, four, five or six adjacent chambers or compartments. If desired, frangible barriers or openable seals (e.g., peel seals or frangible seals) are used to separate the chambers of the multi-chamber container. Multi-chamber containers may also comprise three chambers for accommodating a lipid emulsion, a carbohydrate formulation and an amino acid formulation, and further comprise at least one, in certain embodiments two or three smaller chambers which contain, for example, vitamin formulations and/or trace element formulations. In one specific embodiment, the multi-chamber container of the invention has a first chamber containing the lipid emulsion according to the invention, a second chamber containing an amino acid formulation, a third chamber containing a carbohydrate formulation, a fourth chamber containing a vitamin formulation and a fifth chamber containing a trace element formulation.

A multi chamber container or multi chamber bag (MCB) as used in the context of the invention may be designed for the parenteral administration of its reconstituted content after admixing the formulations contained in the respective chambers. Such MCB may have 2, 3, 4, 5, 6 or more chambers. The chambers of said MCB may have the same size or may have different sizes to accommodate various compositions and volumes. The chambers may be designed to contain volumes of from, for example, 1 to 5 ml, from 5 to 10 ml, from 10 to 50 ml, from 50 to 100 ml, from 100 to 250 ml, from 250 ml to 500 ml, from 500 to 1000 ml, from 1000 to 1500 ml. The MCBs can be designed to have chambers which are located adjacent to each other. The chambers may have various shapes. The chambers can be oriented horizontally and/or vertically to each other. Certain small chambers can be designed to be located within another, larger chamber, wherein, for example, the small chamber which is located within another, larger chamber can be accommodated and fixed into said larger chamber by welding at least one edge of said small chamber in between the weld seam of the surrounding larger chamber.

The openable seals of said multi-chamber containers permit formulations to be separately stored and admixed/reconstituted just prior to administration thereby allowing storage in a single container of formulations which should not be stored as an admixture for an extended period of time. Opening of the seals allows communication between the chambers and mixing of the contents of the respective chambers. The outside seals of the multi-chamber container are strong seals that do not open under the fluid pressure supplied to open the weaker peel seals or frangible seals between the chambers. In some embodiments, the openable seals of the multi-chamber container may be designed to allow for the admixing or reconstitution of only selected chambers of the multi-chamber container, for example, the admixing of the lipid emulsion with the vitamin chamber and the amino acid chamber, if so desired.

The multi-chamber container may be provided with instructions explaining a desired order with which to open the peel seals, so that constituent fluids are mixed in a desired order. The unsealing strengths of the two or more peel seals may be varied to promote the opening of the seals in the desired order. For example, the unsealing strength of the peel seal to be opened first may be 1/3 to 1/2 of the unsealing strength required to open the peel seal to be opened second.

As used herein, amino acid formulations include a sterile, aqueous solution of one or more amino acids and one or more electrolytes. Typically, amino acid formulations include about 2 g to about 20 grams of amino acids per 100 mL of amino acid formulation, such as about 3 grams to about 15 grams and/or about 5 grams to about 15 grams per 100 mL of amino acid formulation. Typical amino acids which are included into amino acid formulations are, for example, isoleucine, leucine, valine, lysine, methionine, phenylalanine, threonine, tryptophan, arginine, histidine, alanine, aspartic acid, cysteine, glutamic acid, glycine, proline, serine, tyrosine, ornithine, and taurine. Further, the content of tyrosine can be increased by adding, for example, a glycyl-tyrosine dipeptide or acetyl-tyrosine (Ac-Tyr). Typically, however, the glycyl-tyrosine dipeptide has improved pharmacokinetics compared to Ac-Tyr, which is more rapidly eliminated by the kidney, resulting in diminished release of tyrosine in the blood.

The amino acid formulation may further include electrolytes. As used herein, electrolytes include sodium, potassium, calcium, magnesium, and/or phosphate ions. For example, the amino acid formulation can include from about 0.1 mmol to about 10 mmol of sodium (e.g., about 3.75 mmol to about 10 mmol of sodium), from about 0.1 mmol to about 10 mmol of potassium (e.g., about 3.75 mmol to about 6.90 mmol of potassium), from about 0.05 mmol to about 1.0 mmol of magnesium (e.g., about 0.05 mmol to about 0.11 mmol and/or about 0.38 mmol to about 0.65 mmol of magnesium), from about 0.1 mmol to about 10 mmol of calcium (e.g., about 1.13 mmol to about 5.10 mmol of calcium), from about 0.1 mmol to about 10 mmol of phosphate (e.g., about 0.94 mmol to about 5.10 mmol of phosphate) and not more than 10 mmol of chloride (e.g., not more than 5.6 mmol of chloride) per 100 mL of amino acid formulation. When calcium and phosphorus are present together in the same heat-sterilized solution, insoluble calcium phosphate precipitation can occur. Accordingly, amino acid formulations may not contain any calcium. Alternatively, by using an organic salt of phosphorus such as sodium glycerophosphate 5·H2O or calcium glycerophosphate, calcium and phosphate amounts may be increased without solubility issues and without providing excess sodium or chloride. In the amino acid formulation, sodium may be provided in the form of sodium chloride, calcium may be provided in the form of calcium chloride 2·H2O or calcium gluconate, magnesium may be provided in the form of magnesium acetate 4·H2O or magnesium chloride, and potassium may be provided in the form of potassium acetate.

Carbohydrate formulations provide a supply of calories, typically in the form of glucose. In particular, the carbohydrate formulation provides an amount of carbohydrate sufficient to avoid adverse effects such as hyperglycemia that has been observed in patients receiving parenteral nutrition. Typically, the carbohydrate formulation includes about 20 to 60 grams of glucose per 100 mL of carbohydrate formulation, such as 25 to 55 grams of glucose per 100 mL of the carbohydrate formulation. Carbohydrate formulations as used in multi-chamber containers for parenteral nutrition according to the present invention may also contain calcium in concentrations from about 0.1 mmol to about 10 mmol of calcium (e.g., about 1.13 mmol to about 5.10 mmol of calcium).

Lipid formulations that serve as a source of lipids as a macronutrient component of the medical product of the invention are an emulsion of an oil phase, a water phase, and an emulsifier that makes the two phases miscible. Furthermore, the medical product comprises a lipid emulsion as claimed herein that comprises vitamin A, D, E and K. The lipid emulsion comprising vitamin A, D, E and K can be comprised by the lipid formulation serving as a major lipid source or can be a different liquid in a different chamber of the medical product.

In the context of the invention, in case of lipid emulsions, which are to be used as an injectable emulsion for parenteral nutrition, the emulsion must be an oil-in-water (o/w) emulsion. This means that the oil must reside in the internal (or dispersed) phase, while water is the external (or continuous) phase, as the emulsion must be miscible with blood. Lipid emulsion as disclosed herein must therefore also be substantially free of any suspended solids. Of course, the lipid emulsions may contain further components, including, but not limited to, antioxidants, pH modifiers, isotonic agents, vitamins, trace elements and various combinations thereof. An overview over lipid emulsions, their composition and use is provided, for example, in Driscoll, Journal of Parenteral and Enteral Nutrition 2017, 41, 125-134. Further information on the use of lipid emulsions in parenteral nutrition of intensive care patients is provided, for example, in Calder et al, Intensive Care Medicine, 2010, 36(5), 735-749.

The oil phase of the lipid emulsion may include polyunsaturated fatty acids, such as long-chain polyunsaturated fatty acids, which may be present as the free acid, as an ionized or salt form of the free acid, and/or in ester form. Suitable esters of the polyunsaturated fatty acids/long-chain polyunsaturated fatty acids include, but are not limited to, alkyl esters (e.g., methyl esters, ethyl esters, propyl esters, or combinations thereof) and triglyceride esters. In some cases, the long-chain polyunsaturated fatty acid has a structure R(C=O)OR', wherein R is an alkenyl group having at least 17 carbon atoms, at least 19 carbon atoms, at least 21 carbon atoms, or at least 23 carbon atoms, and R' is absent, H, a counter ion, an alkyl group (e.g., methyl, ethyl, or propyl), or a glyceryl group (e.g., R(C=O)OR' is a monoglyceride, a diglyceride, or a triglyceride). Polyunsaturated fatty acids for use in the lipid formulations disclosed herein include, but are not limited to, linoleic acid (LA), arachidonic acid (ARA), α-linolenic acid (ALA), eicosapentaenoic acid (EPA), docosahexaenoic acid (DHA), stearidonic acid (SDA), γ-linolenic acid (GLA), dihomo-γ-linolenic acid (DPA), and docosapentaenoic acid (DPA), particularly, DHA, ARA, and EPA, each of which may be present in free acid form, ionized or salt form, alkyl ester form, and/or triglyceride form. In some cases, the polyunsaturated fatty acids and/or long-chain fatty acids are present in triglyceride form.

Typically, the lipid formulation includes about 5% to about 35% by weight of an oil phase based on the total weight of the lipid emulsion. For example, the oil phase of the lipid emulsion is present in an amount of about 8% to 12%, of about 10% to about 20%, of about 10% to about 15%, of about 15% to about 20%, of about 12% to about 17%, of about 18% to 22% and/or about 20% by weight based on the total weight of the lipid formulation. The oil phase typically and preferably contains, in various amounts depending on the source of the oil, omega-3 fatty acids. The three types of omega-3 fatty acids involved in human metabolism are eicosapentaenoic acid (EPA) and docosahexaenoic acid (DHA), both of which are usually found in marine fish oils and α-linolenic acid (ALA), commonly found in plant oils.

The oil phase and its components can be derived from a single source or different sources (see, for example, Fell et al, Advances in Nutrition, 2015, 6(5), 600-610. Of the plant oils, currently used sources include, but are not limited to, soybean and olive oil as well as coconut or palm kernel oil (medium-chain triglycerides (MCTs)). Another source are algae, including microalgae such as Crypthecodinium cohnii and Schizochytrium sp., which in some cases serve as the single source of the long-chain polyunsaturated fatty acid docosahexaenoic acid (DHA). Marine oil used in parenteral lipid emulsions is processed from oily fish primarily found in cold water and including, but not limited to, herring, shad and sardines. However, other marine organisms can be used as an oil source, such as, for example, krill, such as Antarctic krill (Euphausia superba Dana). Krill oil, for example, provides for both EPA and DHA, in amounts of up to 35% w/w of the fatty acids. Krill oil as a component of lipid emulsions is considered to have anti-inflammatory properties due to the presence of DHA and EPA and is hypothesized to bind endotoxins (Bonaterra et al: Krill oil-in-water emulsions protects against lipopoly-saccharides-induced proinflammatory activation of macrophages in vitro. Marine Drugs (2017), 15:74).

The lipid emulsions referred to herein may further include additional components, such as surfactants (also referred to as emulsifiers), co-surfactants, isotonic agents, pH adjusters, and antioxidants. Generally, surfactants are added to stabilize emulsions by reducing the interfacial tension between the oil phase and the aqueous phase. Surfactants typically include a hydrophobic part and a hydrophilic part, and the amount of surfactant/emulsifier included in the formulations is determined based on the amount that is needed to achieve a desired level of stabilization of the emulsion. Typically, the amount of surfactant in the lipid formulation is about 0.01 % to about 3% by weight based on the total weight of the lipid formulation, for example, about 0.01 % to about 2.5%, about 0.01 % to about 2.3%, about 0.02 % to about 2.2%, about 0.02 % to about 2.1%, about 0.02 % to about 2%, about 0.05% to about 1.8%, about 0.1% to about 1.6%, about 0.5% to about 1.5%, about 0.8% to about 1.4%, about 0.9% to about 1.3%, about 1% to about 1.2%, and/or about 1.2% by weight. Suitable surfactants and co-surfactants include surfactants that are approved for parenteral use, and include, but are not limited to, phospholipids (e.g., egg phosphatide and soy lecithin), oleate salts, and combinations thereof. Krill oil can also be used as an emulsifier in the lipid emulsion, wherein the lipid emulsion comprises about 0.5 to 2.2 wt % krill oil based on the total weight of the emulsion, and wherein the emulsion is free of egg yolk lecithin (US 2018/0000732 A1). Another exemplary surfactant is lecithin, including both natural and synthetic lecithin, such as lecithins derived from egg, corn or soybean or mixtures there-of. In some cases, lecithin is included in an amount of about 1.2% based on the total weight of the lipid formulation.

In embodiments, the lipid emulsions of the medical product of the invention, in particular the lipid emulsion comprising vitamins A, D, E and K comprise as a lipid component a low peroxide level oil, preferably selected from the group consisting of soybean oil with low peroxides, olive oil, medium chain triglycerides, fish oil, fish oil extract, krill oil, algae oil, safflower oil, sunflower oil, corn oil, coconut oil, palm kernel oil, rapeseed oil, fungal oil and hydrogenated oils or mixtures thereof.

In embodiments, a lipid or oil with a low peroxide level has a peroxide value of no more than 5 milliequivalents (mEq) O₂/kg, preferably no more than 3 mEq O₂/kg, more preferably no more than 1.5 mEq O₂/kg. The oils listed above have been described as complying with this requirement. Furthermore, a person skilled in the art is able to identify low peroxide oils or lipids, such as oils with a peroxide value of no more than 5 milliequivalents (mEq) O₂/kg, by determining the peroxide value.

The peroxide value gives a measure of the extent to which an oil has undergone primary oxidation. The extent of secondary oxidation may be determined from p-anisidine test. The double bonds found in fats and oils play a role in autoxidation. Oils with a high degree of unsaturation are most susceptible to autoxidation. The best test for autoxidation (oxidative rancidity) is determination of the peroxide value. Peroxides are intermediates in the autoxidation reaction. Autoxidation is a free radical reaction involving oxygen that leads to deterioration of fats and oils. The peroxide value is defined as the amount of peroxide oxygen per 1 kilogram of fat or oil. Traditionally this was expressed in units of milliequivalents, and the SI unit is in millimoles per kilogram (N.B. 1 milliequivalents = 0.5 millimole; because 1 mEq of O₂ =1 mmol/2=0.5 mmol of O₂, where 2 is valence).

The peroxide value is determined by measuring the amount of iodine which is formed by the reaction of peroxides (formed in fat or oil) with iodide ion.

2 I⁻ + H₂O + HOOH -> HOH + 2OH⁻ + I₂

The base produced in this reaction is taken up by the excess of acetic acid present. The iodine liberated is titrated with sodium thiosulphate.

2S₂O₃²⁻ + I₂ -> S₄O₆²⁻ + 2 I⁻

The acidic conditions (excess acetic acid) prevents formation of hypoiodite (analogous to hypochlorite), which would interfere with the reaction. The indicator used in this reaction is a starch solution where amylose forms a blue to black solution with iodine and is colourless where iodine is titrated. A precaution that should be observed is to add the starch indicator solution only near the end point (the end point is near when fading of the yellowish iodine colour occurs) because at high iodine concentration starch is decomposed to products whose indicator properties are not entirely reversible. Peroxide values of fresh oils are less than 10 milliequivalents O₂/kg; when the peroxide value is between 30* and 40 milliequivalents O₂/kg, a rancid taste is noticeable. As used herein, a low peroxide oil is an oil with no more than 5 milliequivalents O₂/kg, preferably no more than 3 mEq O₂/kg, more preferably no more than 1.5 mEq O₂/kg.

In some cases, the lipid emulsion formulation includes a co-surfactant. Typically, the amount of co-surfactant in the lipid formulation is less than the amount of surfactant, and typically the amount of co-surfactant in the formulation is about 0.001% to about 0.6% by weight based on the total weight of the lipid formulation, for example, about 0.001% to about 0.55%, about 0.001% to about 0.525%, about 0.001% to about 0.5%, about 0.005% to about 0.5%, about 0.01% to about 0.4%, about 0.02% to about 0.3%, about 0.03% to about 0.2%, about 0.04% to about 0.1%, and/or about 0.05% to about 0.08%. An exemplary co-surfactant is oleate, such as sodium oleate. In some cases, the lipid formulation includes lecithin and oleate as surfactant and co-surfactant, for example, an in amount of 1.2% lecithin and 0.03% oleate. In some cases, sodium oleate is included in an amount of about 0.03% by weight based on the total weight of the lipid formulation.

Isotonic agents can be added to the lipid emulsions to adjust the osmolarity of the lipid emulsion to a desired level, such as a physiologically acceptable level. Suitable isotonic agents include, but are not limited to, glycerol. Typically, the lipid emulsion formulation has an osmolarity of about 180 to about 300 milliosmole/liter, such as about 190 to about 280 milliosmole/liter, and/or about 200 to about 250 milliosmole/liter. In some cases, the lipid emulsion includes an isotonic agent in an amount of about 1% to about 10% by weight based on the total weight of the lipid formulation, such as about 1% to about 5%, about 1% to about 4%, and/or about 2% to about 3%. In some cases, the lipid emulsion formulation includes about 2% to about 3% by weight of glycerol.

pH modifiers can be added to the lipid emulsions to adjust the pH to a desired level, such as a physiologically acceptable pH for parenteral use. Suitable pH modifiers include but are not limited to sodium hydroxide and hydrochloric acid. Typically, the lipid emulsions of the invention, in particular the lipid emulsion comprising vitamin A, D, E and K has a pH of about 5 to about 9, such as about 5.1 to about 8.9, about 5.2 to about 8.8, about 5.3 to about 8.7, about 5.4 to about 5.6, about 5.5 to about 5.5, about 5.6 to about 8.4, about 5.7 to about 8.3, about 5.8 to about 8.2, about 5.9 to about 8.1, about 6 to about 8, about 6.1 to about 7.9, about 6.2 to about 7.8, about 6.3 to about 7.7, about 6.4 to about 7.6, about 6.5 to about 7.5, about 6.6 to about 7.4, about 6.7 to about 7.3, about 6.8 to about 7.2, about 6.9 to about 7.1, and/or about 7.

The lipid formulation and/or lipid emulsion comprising vitamin A and optionally vitamins D, E and K may further include antioxidants. Suitable antioxidants may be pharmaceutically acceptable antioxidants and include, but are not limited to, EDTA, tocopherols (e.g., gamma tocopherol, delta tocopherol, alpha tocopherol), ascorbyl palmitate, butylhydroxytoluol (BHT) or combinations thereof. In some cases, the lipid emulsions can include an antioxidant in an amount of about 0 to about 200 mg/L, for example, about 10 to about 200 mg/L, about 40 to about 150 mg/L, about 50 to about 120 mg/L, about 75 to about 100 mg/L antioxidant (s), such as vitamin E.

The aqueous (or water) phase of all intravenous lipid emulsions must conform to the pharmacopeial requirements that make it suitable for injection, that is, the water must be sterile water for injection.

The lipid emulsions can be prepared according to generally known processes (see, for example, Hippalgaonkar et al, AAPS PharmSciTech 2010, 11(4), 1526-1540 or WO 2019/197198 A1)). Generally, water-soluble and oil-soluble ingredients are dissolved in the aqueous phase and oil phase, respectively.

If not defined otherwise herein, all terms used in the context of the present invention are to be interpreted according to the understanding of a person skilled in the art.

### FIGURES

The invention is further described by the following figures. These are not intended to limit the scope of the invention but represent preferred embodiments of aspects of the invention provided for greater illustration of the invention described herein.

### Description of the Figures:

**Figure 1****:** Examples of compounds comprised by the term vitamin A comprised by the present invention.
**Figure 2****:** The graph shows the domain of stability (white area) of vitamin A. Stability is defined as a recovery of from 80% to 120%. The hatched area shows where vitamin A is not stable any more in dependency on oxygen levels over time. Accordingly, at an oxygen level of about 0.300 ppm vitamin A after 6 months is no longer considered stable, i.e. recovery drops below 80%.

### EXAMPLES

The invention is further described by the following examples. These are not intended to limit the scope of the invention but represent preferred embodiments and/or certain aspects of the invention provided for greater illustration of the invention described herein.

### Example 1: Methods for determining vitamin A concentration

Determination of fat-soluble vitamins can be accomplished by Ultra Performance Liquid Chromatography (UPLC), which separates components of a solution by partitioning of the analytes between a mobile liquid phase and a liquid stationary phase (reversed phase mode). The resulting difference in elution time allows to independently detect the different analytes. It is also possible to determine vitamin A by using HPLC with UV, fluorescence (FLD), electrochemical (ED), or evaporative light scattering (ELSD) detection methods. Generally, care should be taken to reduce exposure of the samples to light and use the respective light-protective equipment. For example, Vitamin A concentrations can be determined by high-performance liquid chromatography (HPLC) as described by Lee et al., Simultaneous Determination of Vitamin A and E in Infant Formula by HPLC with Photodiode Array Detection, Korean J Food Sci Ani Resour 31(2) 191-199. The standard method for the quantitative determination of vitamin a comprises saponification of the test material with aqueous ethanolic potassium hydroxide, and the liberation of vitamin A by extraction with n-hexane. After concentration of the extract, the residue is dissolved in methanol and the vitamin A content is determined after HPLC separation on a RP-C₁₈ column by means of an UV or fluorescence detector.

### Example 2: Methods for determining the concentration of dissolved oxygen (DO)

Various methods can be used to determine the DO content of a given solution. Generally, electrochemical or optical sensors are used for determining the DO concentration according to the invention. A preferred method comprises using a fiber-optic oxygen meter such as MICROX TX3, which is a single-channel, temperature-compensated oxygen meter with fiber-optic trace oxygen microsensors based on a glass fiber (e.g. 140 µm). The optical oxygen microsensors (also called optodes) do not consume oxygen, their signal does not depend of the flow rate of the sample, the diameter of the microsensor tip is e.g. < 50 µm, they measure oxygen in both liquid and gas phase and they have an on-line temperature compensation of the oxygen content. For data visualization during measurements, the oxygen meter is connected to a LCD & Data-Logger Unit.

The oxygen measure and adjustment are based on an equilibrium between exposure to nitrogen and oxygen containing gases during the mixing and filling steps of the process. An acute and regular monitoring of the solution DO throughout these process steps allows to control the oxygen level within the final container. Besides, the headspace of the bag can be filled with a specific volume of nitrogen or oxygen to reach the final targeted level of DO.

### Example 3: Recovery of vitamin A from mixed micelle and lipid emulsion formulation under different conditions

Stability of vitamin A in terminally heat-sterilized compositions such as lipid emulsions and solutions comprising mixed micelles have been examined in laboratory experiments. Multiple studies have been performed, as summarized in **Table 4.**

**Table 4. Summary of studies and experiments performed in order to optimize the composition of the lipid emulsion comprising vitamin A to be comprised in a medical product of the invention.**

| **Formulation study** | **Pharmaceutical form** | **Formulation comprising** | **Container** | **Process** | **Outcome, Recovery of Vitamin A** |
|---|---|---|---|---|---|
| Study A | Mixed micelles | A/D3/E/K sorbitol | Non-PVC, non-oxygen barrier bags | Terminal heat sterilization | 26.2 -27.2 % recovery 6 months at 25°C |
| Study A1 | Mixed micelles | A/D/E/K sorbitol citric acid | Non-PVC, non-oxygen barrier bags | Terminal heat sterilization | 26.8 -26.9 % recovery 6 months at 25°C |
| Study A2 | Mixed micelles | A/D/E/K sorbitol | Non-PVC, non-oxygen barrier bags | Non sterilized | 56.6 -90.6 % recovery 6 months at 25°C |
| Study A3 | Mixed micelles | A/D/E/K sorbitol / citric acid | Non-PVC, non-oxygen barrier bags | Not sterilized | 57.5 -58.3 % recovery 6 months at 25°C |
| **Study B1** | **Emulsion** | **A/D/E/K 20% lipid emulsion; (80% olive oil, 20% soybean oil) ascorbyl palmitate** | **Non-PVC oxygen barrier bags** | **Terminal heat sterilization** | **77.0-78.7% recovery 6 months at 40°C** |
| Study B2 | Mixed micelles | A/D/E/K ascorbyl palmitate | Non-PVC oxygen barrier bags | Terminal heat sterilization | 31.6-36.6 % recovery 1 month at 40°C |
| Study B3 | Micellar solution | A/D/E/K Polysorbate 80 & 20 PEG 400 sorbitol | Non-PVC oxygen barrier bags | Terminal heat sterilization | 30.2 - 33.8 % recovery 1 month at 40°C |
| **Study C1** | **Lipid emulsion** | **A/D/E/K 10% lipid emulsion; soybean oil ascorbyl palmitate** | **Non-PVC oxygen barrier bags +oxygen absorber** | **Terminal heat sterilization** | **95% recovery 3 months at 40°C** |
| **Study C2** | **Lipid emulsion** | **A/D/E/K 20% lipid emulsion; soybean oil ascorbyl palmitate** | **Non-PVC, non-oxygen barrier bags** | **Terminal heat sterilization** | **90% recovery 3 months at 40°C** |
| **Study C3** | **Lipid emulsion** | **A/D/E/K 5% lipid emulsion; olive oil ascorbyl palmitate** | **Non-PVC, non-oxygen barrier bags + oxygen absorber** | **Terminal heat sterilization** | **90% recovery 3 month at 40°C** |
| **Study D1** | **Lipid emulsion** | **A/D/E/K 10% lipid emulsion; soybean oil** | **Non-PVC, non-oxygen barrier bags + oxygen absorber** | **Terminal heat sterilization** | **78.0 - 86.0% recovery 6 months at 40°C** |

The experiments were performed with vitamin formulations comprising combinations of vitamins A, D, E and K. The results indicated that recovery rates for vitamin A are favorable especially in lipid emulsions, such as, for example, a 10% lipid emulsion with soybean or olive oil or mixtures of these oils, see also Study C1. However, 5% emulsions (e.g. Study C3) as well as 20% emulsions (Study C2) also gave good results, especially considering that the formulations were stored in non-oxygen barrier bags on the latter cases. Therefore, lipid emulsions in the range of from 5% to 20% oil phase in a range of from 5% to 20% w/v of an oil are recommendable for formulations for stably accommodating vitamin A. In addition, using oxygen barrier bags and potentially also oxygen absorbers further improve the stability and thus recovery of vitamin A after storage (Study C1).

In contrast, mixed micelle formulations could not provide for a reasonable stability of vitamin A, even in the presence of oxygen barrier bags (Study B2, B3) and in the presence of various antioxidants, such as, for example, sorbitol, citric acid or ascorbyl palmitate, and where the mixed micelle formulation was not terminally heat-sterilized (Studies A2 and A3). Using oxygen-barrier bags increased the stability of vitamin A (Study B1) but could not bring it to levels obtained with lipid emulsions in oxygen-barrier bags.

Using oxygen barrier bags clearly further improved the stability of vitamin A. Accordingly, it could be confirmed that oxygen levels play an important role in the stability of vitamin A. This was further investigated in **Example 4.**

### Example 4: Impact of oxygen on the stability of vitamin A

The stability of vitamin A was tested in a 10% soybean lipid emulsion having a peroxide level of below 5 milliequivalents (mEq) O₂/kg. The lipid emulsion contained vitamin A in a concentration of 1dd/25mL, as well as vitamin E in a concentration of 1dd/25 mL and the pH was adjusted to 5.9 with HCl.

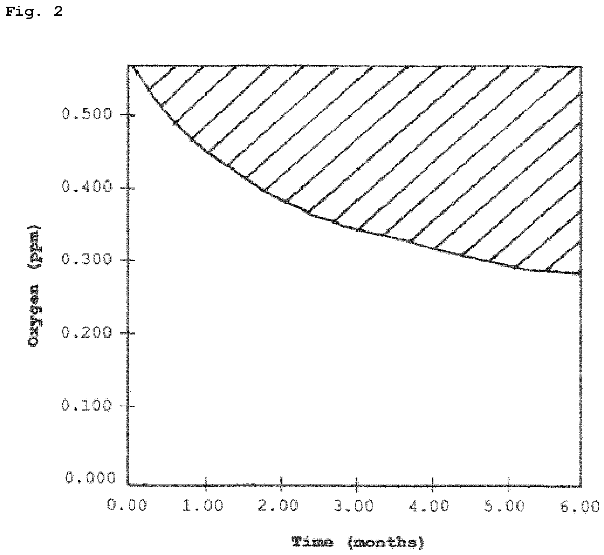

**Figure 2** depicts the dependency of vitamin A recovery on the presence of dissolved oxygen. Independently of the light exposure, it was found that vitamin A is not stable in the presence of oxygen of above 0.3ppm for more than 6 months at 40°C. Specifically, it was found that for a recovery of at least 80% after 6 months at 40°C, as indicated in Figure 2, DO level of less than about 0.3 ppm is required. In contrast, no such dependency could be found for vitamin E, which underlines the need to carefully adjust the conditions under which vitamin A is formulated into a parenteral nutrition formulation, specifically in a large volume container such as a multi-chamber container which should also be terminally heat-sterilized.

### Example 5: Impact of heat exposure on the stability of vitamin A

A 10% lipid emulsion based on soybean oil containing vitamin A at a concentration of 1dd/25 ml was provided in a flexible container having a volume of 25 mL at a native pH of about 7.5-8.0. The container consisted of a non-oxygen barrier material. The container was overpouched with a light protective material and an oxygen absorber was added, meaning that no residual oxygen remains in the formulation. The formulation was then submitted to different sterilization conditions. The recovery of vitamin A after sterilization and storage for 6 months at 40°C was assessed. The results are summarized in **Table 5.** The C₀ value is a measure of the total heat exposure of the API vitamin A. The results demonstrate that vitamin A is susceptible to the total heat exposure it is submitted to, which is a critical component for producing a product according to the invention, especially when the vitamin A formulation forms part of a multi-chamber container having a large volume, such as a 2-, 3-, four- or five-chamber container which, when terminally heat sterilized, requires a significantly higher heat exposure to arrive at a required Fo compared to, for example, a small container of less than, for example, 100 mL, such as a 25 mL or 50 mL container, where also light exposure and oxygen levels can be controlled much more easily.

**Table 5: Effect of heat on the stability of vitamin A. The heat exposure of the compound is provided as Co in minutes, which gives a measure for the total heat exposure to which vitamin A is subjected. TO represents the recovery of vitamin A before sterilization. Recovery rates after storage for 6 months at 40°C show that the impact of steam sterilization on the vitamin A stability is more pronounced over longer storage times. Obviously, a higher heat exposure during sterilization has a long-term negative effect on vitamin A stability.**

| **Sterilization Method** | **Steam sterilization** | **Water cascade sterilization** | |
|---|---|---|---|
| **c0 (min.)** | 77 | 49 | 37 |
| **T0** | 104% | 96% | 97% |
| **T6M40°C** | 78% | 82% | 86% |

### Example 6: Impact of pH on the stability of vitamin A

Two batches of a vitamin A containing formulation as shown in **Table 6** were tested regarding the impact of pH on the stability of vitamin A. Both batches were formulated as 5% lipid emulsions with olive oil and stored in an oxygen-barrier material flexible container (overpouched with light protective material and with oxygen absorber) having a volume of 25 mL.

**Table 6: Effect of pH on vitamin A stability after sterilization and storage at 40°C for 6 months. TO refers to the recovery rate before sterilization.**

| | **Batch 1** | **Batch 2** |
|---|---|---|
| **Emulsion type** | 5% emulsion (olive oil) | 5% emulsion (olive oil) |
| **Vitamins concentration** | 1dd/25 mL | 1dd/25 mL |
| **Bag material** | Oxygen-barrier | Oxygen-barrier |
| **pH** | **Native (7.5-8)** | **Adjusted (5.9)** |
| **T0 unsterilized** | 100% | 100% |
| **T6M40°C sterilized** | 73% | 87.5% |

Surprisingly, the recovery rate for vitamin A improves when adjusting the pH to a pH which is below the native one at about 7.5 to 8.0. Accordingly, the pH contributes together with other parameters to the stability of vitamin A in a product according to the invention, specifically also when the vitamin A formulation forms part of a terminally heat-sterilized multi-chamber container having a high volume.

## Claims

1. A medical product for preventing or correcting vitamin A deficiency in a patient comprising a lipid emulsion in a flexible container, the lipid emulsion comprising:
a. vitamin A in the lipid phase of the lipid emulsion,
b. optionally additionally vitamin D, vitamin E and/or vitamin K in the lipid phase of the lipid emulsion, and
c. no more than 1.5 ppm dissolved oxygen (DO),
d. wherein the pH of the lipid emulsion is from 5 to 9.

2. The medical product according to the preceding claim, wherein the vitamin A is present in form of retinyl palmitate, retinyl acetate, retinal, retinol and/or retinoic acid, preferably retinyl palmitate.

3. The medical product according to any one of the preceding claims, wherein the lipid emulsion comprises as a lipid component a low peroxide level oil, preferably selected from the group consisting of soybean oil with low peroxides, olive oil, medium chain triglycerides, fish oil, fish oil extract, krill oil, algae oil, safflower oil, sunflower oil, corn oil, coconut oil, palm kernel oil, rapeseed oil, fungal oil and hydrogenated oils or mixtures thereof.

4. The medical product according to any one of the preceding claims, wherein the lipid emulsion comprises as a lipid component an oil with a peroxide value of no more than 5 milliequivalents (mEq) O₂/kg, preferably no more than 3 mEq O₂/kg, more preferably no more than 1.5 mEq O₂/kg.

5. The medical product according to any one of the preceding claims, wherein the lipid emulsion has a lipid concentration of 2-40 %, preferably 5-30 %, more preferably 5-20 %.

6. The medical product according to any one of the preceding claims, wherein the lipid emulsion comprises an antioxidant agent, preferably selected from the group consisting of EDTA, tocopherol, ascorbyl palmitate and butylhydroxytoluol (BHT).

7. The medical product according to any one of the preceding claims, wherein the medicinal product comprises 2000 - 4000 International Units (IU) vitamin A.

8. The medical product according to any one of the preceding claims, wherein vitamin A is present in the emulsion at a concentration of at least 20 IU vitamin A per gram of lipid (IU/gram), preferably at a concentration of 20 to 80.000 IU/gram.

9. The medical product according to any one of the preceding claims, wherein the lipid emulsion further comprises vitamins selected from the group consisting of vitamins B2, B3, B5, B8, B9 and B12, wherein preferably the lipid emulsion further comprises vitamins B2, B5 and B12.

10. The medical product according to any one of the preceding claims, wherein the medicinal product comprises a light-protective outer wrapping.

11. The medical product according to any one of the preceding claims, wherein the flexible bag comprises an inner lining containing a polyolefin, wherein the inner lining is free of polyvinyl chloride (PVC), plasticizers, adhesives or latex.

12. The medical product according to any one of the preceding claims, wherein the vitamin A is stable in the lipid emulsion for at least three months when stored at a temperature between 1 and 50 °C.

13. The medical product according to any one of the preceding claims, wherein the vitamin A is stable in the lipid emulsion for at least 6 months, preferably 12 months, more preferably 18 months, most preferably 24 months at a temperature of from 1°C to 32°C, preferably from 1°C to 25°C.

14. The medical product according to any one of the preceding claims, wherein the lipid emulsion is a sterilized lipid emulsion.

15. The medical product according to any one of the preceding claims, wherein the lipid emulsion does not comprise macronutrients selected from the group consisting of carbohydrates and proteins, and wherein preferably the solution does not comprise any nutrients besides lipids and vitamins.

16. The medical product according to any one of the preceding claims, wherein the emulsion and medical product are configured for parenteral administration.

17. A medical product according to any of the preceding claims, wherein the lipid emulsion is contained in one chamber of a multi chamber container having at least two, at least three, at least four, at least five or at least six chambers.

18. The medical product according to claim 17, wherein the container comprises at least a first chamber containing a carbohydrate formulation, a second chamber containing an amino acid formulation, a third chamber containing a lipid formulation, and optionally comprises electrolytes and/or vitamins and/or trace elements in the first, second and/or third chamber.

19. The medical product according to claim 17 or 18, wherein the lipid emulsion is present in a fourth chamber.

20. The medical product according to claim 17 to 19, wherein the lipid emulsion is present in a chamber comprising a lipid formulation.

21. A method of preparing a medical product for preventing or correcting vitamin A deficiency in a patient according to any one of the preceding claims, wherein the lipid emulsion comprises a lipid phase and an aqueous phase and is prepared by the steps comprising
a. Separately heating up the lipid phase and the aqueous phase to a temperature of from 60°C to 90°C under agitation;
b. Adding vitamin A, and optionally vitamin D, vitamin E and/or vitamin K to the lipid phase;
c. Preparing the pre-emulsion by transferring the lipid phase to the aqueous phase under agitation;
d. Homogenizing the pre-emulsion at a temperature of from 40°C to 60°C under pressure;
e. Optionally adding water to adjust the required volume and concentrations;
f. Adjusting the lipid emulsion to a concentration of dissolved oxygen (DO) of no more than 1.5 ppm and a pH value of 5-9, and
g. Sterilizing the solution, preferably by terminal heat sterilization, more preferably by terminal heat sterilization with moist heat.

22. A sterilized lipid emulsion for parenteral administration for use in preventing or correcting vitamin A deficiency, comprising
a. vitamin A, preferably in the form of retinyl palmitate, in a lipid phase of a lipid emulsion,
b. optionally vitamin D, vitamin E and/or vitamin K in the lipid phase of the lipid emulsion,
c. no more than 1.5 ppm DO, and with a pH of 5-9.
